# EUROPEAN PATENT APPLICATION

(11) **EP 4 755 883 A2**
(43) Date of publication of application: **10.06.2026**
(21) Application number: 26165233.3
(22) Date of filing: 29.12.2023
(51) Int. Cl.: C07D 457/06

(54) **SALTS OF 2-BROMOLYSERGIC ACID DIETHYLAMIDE**

(30) Priority: 31.12.2022 US 202263436534 P
(62) Divisional of application: 23848757.3
(71) Applicant: Ceruvia Lifesciences LLC, Greenwich, CT 06830 (US)
(72) Inventor: MOORE, Gillian, Durham, TS21 2JB (GB); NORTHEN, Julian S., South Shields, NE33 4TH (GB)
(74) Representative: HGF

(57) **Abstract**

The present disclosure relates to salts of 2-bromolysergic acid diethylamide (2-Br-LSD), pharmaceutical compositions thereof, and methods of preparing and using the 2-Br-LSD salts.

## Description

### RELATED APPLICATIONS

This application claims the benefit of priority under 35 U.S.C. § 119(e) to U.S. Provisional Application No: 63/436,534, filed December 31, 2022, which is incorporated herein by reference in its entirety.

### BACKGROUND OF THE DISCLOSURE

The compound 2-bromolysergic acid diethylamide (2-Br-LSD) (also known as BOL-148) is a nontoxic second-generation LSD-derived molecule that mimics the therapeutic potential of LSD, for example, cluster headaches and migraines, without the psychedelic effects or hallucinations (Karst et al., Cephalalgia 30(9):1140-4 (2010)). 2-Bromolysergic acid diethylamide is currently in phase 1 clinical trials for migraines and cluster headaches (Turnbull, C., (2021, August 11), Ceruvia Lifesciences Drug Trials Work To Turn Psychedelic Research Into Medicine for OCD, Headache Disorders, AP NEWS, Release ID: 89040284).

### SUMMARY OF THE DISCLOSURE

A first aspect of the present disclosure is directed to crystalline salts of 2-bromolysergic acid diethylamide (2-Br-LSD), also known as NYPRG-101. The salts include hydrobromide, hemi-fumarate, mesylate, and besylate salts.

In some embodiments, the crystalline salt of the present disclosure is 2-Br-LSD hydrobromide.

In some embodiments, the 2-Br-LSD crystalline salt (2-Br-LSD hydrobromide (Form 1)) has an XRPD pattern comprising the following 2Θ peaks measured using CuK_{α} radiation: 8.0 ± 0.2, 14.2 ± 0.2, 21.9 ± 0.2, 23.3 ± 0.2, 28.6 ± 0.2, 30.4 ± 0.2. In some embodiments, the 2-Br-LSD crystalline salt (2-Br-LSD hydrobromide (Form 1)) has an XRPD pattern further comprising the following 2Θ peaks measured using CuK_{α} radiation: 12.5 ± 0.2, 17.8 ± 0.2, 19.0 ± 0.2, 24.0 ± 0.2, 24.5 ± 0.2. In some embodiments, the 2-Br-LSD crystalline salt (2-Br-LSD hydrobromide (Form 1)) has an XRPD pattern even further comprising the following 2Θ peaks measured using CuK_{α} radiation: 26.1 ± 0.2, 26.9 ± 0.2.

Another aspect of the present disclosure is directed to a pharmaceutical composition comprising the crystalline salt, and a pharmaceutically acceptable carrier.

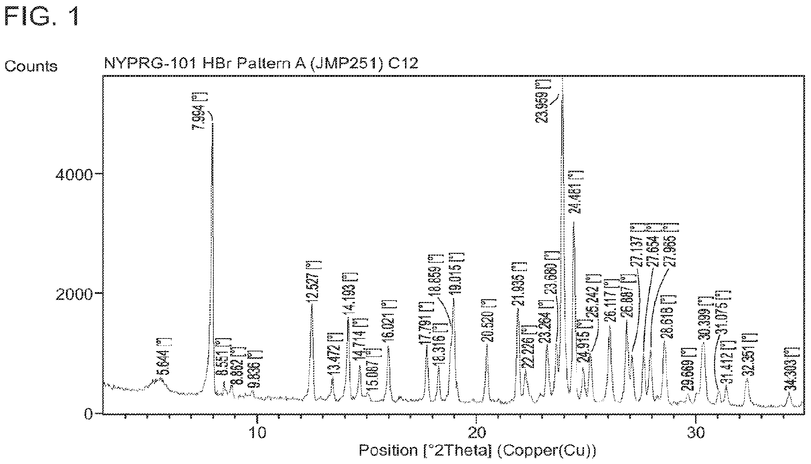

In some embodiments, the pharmaceutical composition is in the form of a solid or a liquid. Another aspect of the present disclosure is directed to a method of preparing crystalline 2-Br-LSD hydrobromide (Form 1), which has an XRPD pattern substantially similar to that set forth in FIG. 1 as measured using CuK_{α} radiation, comprising the following steps:
(a) equilibrating a solution comprising 2-Br-LSD and a solvent at a first temperature;
(b) adding a first portion of hydrobromic acid to the first-temperature equilibrated solution;
(c) equilibrating the batch at the first temperature for a period of time M;
(d) adding a second portion of hydrobromic acid to the batch over a period of time N;
(e) equilibrating the batch at the first temperature for a period of time O;
(f) cooling the batch to a second temperature;
(g) equilibrating the batch at the second temperature for a period of time P;
(h) isolating precipitate from the suspension; and
(i) drying the isolated precipitate *in vacuo* at a third temperature to obtain the crystalline salt;
   wherein M is between 0 and 24 hours;
   wherein N is between 5 minutes and 24 hours;
   wherein O is between 0 and 8 hours;
   wherein P is between 30 minutes and 48 hours.

### BRIEF DESCRIPTION OF THE DRAWINGS

**FIG.** 1 is an X-ray powder (XRP) diffractogram of 2-Br-LSD hydrobromide (Form 1).
FIG. 2 is a graph of differential scanning calorimetry (DSC) and thermal gravimetric analysis (TGA) curves of 2-Br-LSD hydrobromide (Form 1).
FIG. 3 is a ¹H NMR spectrum (400 MHz) of 2-Br-LSD hydrobromide.
FIG. 4 is an XRP diffractogram of 2-Br-LSD hydrobromide (Pattern B Solvate).
FIG. 5 is an XRP diffractogram of 2-Br-LSD hydrobromide (Form 2).
FIG. 6 is an XRP diffractogram of 2-Br-LSD hemi-fumarate (Form A).
FIG. 7 is an XRP diffractogram of 2-Br-LSD mesylate (Form A).
FIG. 8 is an XRP diffractogram of 2-Br-LSD besylate.
FIG. 9 is a ¹H NMR spectrum (400 MHz) of 2-Br-LSD mesylate.
FIG. 10 is a dynamic vapor sorption isotherm (25 °C) of 2-Br-LSD hydrobromide (Form 1).
FIG. 11 is a comparison of the XRPD patterns of 2-Br-LSD hydrobromide (Form 1) (top) ex DVS following equilibration at 0% relative humidity (middle) and 90% relative humidity (bottom).
FIG. 12 is a ¹H NMR spectrum (400 MHz) of 2-Br-LSD oxalate.
FIG. 13 is a ¹H NMR spectrum (400 MHz) of 2-Br-LSD hemi-fumarate.
FIG. 14 is a ¹H NMR spectrum (400 MHz) of 2-Br-LSD mono-succinate.
FIG. 15 is a graph of DSC and TGA curves of 2-Br-LSD besylate.
FIG. 16 is a ¹H NMR spectrum (400 MHz) of 2-Br-LSD xinafoate.
FIG. 17 is a graph of DSC and TGA curves of 2-Br-LSD mono-succinate (Form A)
FIG. 18 is an XRP diffractogram of 2-Br-LSD mono-succinate (Form A)
FIG. 19 is a ¹H NMR spectrum (300 MHz) of 2-Br-LSD hydrobromide.
FIG. 20 is a ¹H NMR spectrum (300 MHz) of 2-Br-LSD hydrobromide, after D₂O exchange.
FIG. 21 is a ¹³C NMR spectrum of 2-Br-LSD hydrobromide.
FIG. 22 is a ¹³C NMR spectrum of 2-Br-LSD hydrobromide, expanded from about 10 ppm to about 70 ppm.
FIG. 23 is a mass spectrum of 2-Br-LSD hydrobromide.
FIG. 24 is an XRP diffractogram of 2-Br-LSD hydrobromide (Form 1).
FIG. 25 is a DSC curve of 2-Br-LSD hydrobromide (Form 1).
FIG. 26 is a ¹H NMR spectrum (400 MHz) of 2-Br-LSD hydrobromide.
FIG. 27 is an XRP diffractogram of 2-Br-LSD hydrobromide (Form 1).
FIG. 28 is a DSC curve of 2-Br-LSD hydrobromide (Form 1).
FIG. 29 is a TGA curve of 2-Br-LSD hydrobromide (Form 1).
FIG. 30 is a microscopy image of 2-Br-LSD hydrobromide x50 objective dispersed in oil.
FIG. 31 is an XRP diffractogram of 2-Br-LSD mesylate (Form B).
FIG. 32 is a graph of DSC and TGA curves of 2-Br-LSD mesylate (Form B).
FIG. 33 is an XRP diffractogram of 2-Br-LSD mesylate (Form C).
FIG. 34 is a graph of DSC and TGA curves of 2-Br-LSD mesylate (Form C).
FIG. 35 is a ¹H NMR spectrum of 2-Br-LSD mesylate.
FIG. 36 is a graph of DSC and TGA curves of 2-Br-LSD hemi-fumarate (Form A).
FIG. 37 is a graph of DSC and TGA curves of 2-Br-LSD hemi-fumarate (Form B).
FIG. 38 is an XRP diffractogram of 2-Br-LSD hemi-fumarate (Form B).
FIG. 39 is a graph of DSC and TGA curves of 2-Br-LSD oxalate (Form A).
FIG. 40 is an XRP diffractogram of 2-Br-LSD oxalate (Form A).
FIG. 41 is a graph of DSC and TGA curves of 2-Br-LSD xinafoate (Form A).
FIG. 42 is an XRP diffractogram of 2-Br-LSD xinafoate (Form A).
FIG. 43 is an XRP diffractogram of 2-Br-LSD xinafoate (Form B).
FIG. 44 is a graph of DSC and TGA curves of 2-Br-LSD hemi-succinate (Form A).
FIG. 45 is an XRP diffractogram of 2-Br-LSD hemi-succinate (Form A).

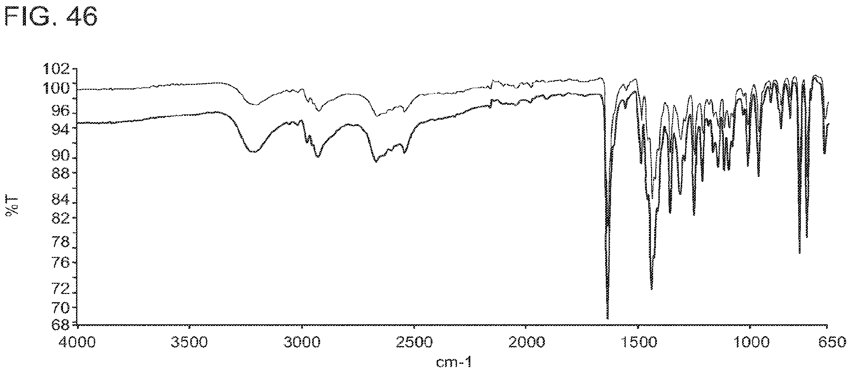

FIG. 46 is a Fourier transform infrared spectrum of 2-bromolysergic acid diethylamide hydrobromide (Form 1) prepared according to Example 2, Method 4 (lower trace) overlayed with that of the reference standard (upper trace) prepared and characterized according to Example 2, Method 3.
FIG. 47 is an X-ray powder (XRP) diffractogram of 2-bromolysergic acid diethylamide hydrobromide (Form 1) prepared according to Example 2, Method 4.

### DETAILED DESCRIPTION

Unless defined otherwise, all technical and scientific terms used herein have the same meaning as is commonly understood by one of skill in the art to which the subject matter herein belongs. As used in the specification and the appended claims, unless specified to the contrary, the following terms have the meaning indicated in order to facilitate the understanding of the present disclosure.

As used in the description and the appended claims, the singular forms "a", "an", and "the" include plural referents unless the context clearly dictates otherwise. Thus, for example, reference to "a composition" includes mixtures of two or more such compositions, reference to "an inhibitor" includes mixtures of two or more such inhibitors, and the like.

Unless stated otherwise, the terms "about" and "substantially" (as in "substantially similar") mean within 10% (e.g., within 5%, 2% or 1%) of the particular value modified by the terms "about" and "substantially."

The transitional term "comprising," which is synonymous with "including," "containing," or "characterized by," is inclusive or open-ended and does not exclude additional, unrecited elements or method steps. By contrast, the transitional phrase "consisting of" excludes any element, step, or ingredient not specified in the claim. The transitional phrase "consisting essentially of" limits the scope of a claim to the specified materials or steps "and those that do not materially affect the basic and novel characteristic(s)" of the claimed invention.

The term "crystalline" refers to a solid form that is substantially non-amorphous. In certain embodiments, a crystal form of a salt described herein may be substantially free of amorphous solids and/or other crystal forms. In certain embodiments, a crystal form of a compound described herein may contain less than about 1%, less than about 2%, less than about 3%, less than about 4%, less than about 5%, less than about 6%, less than about 7%, less than about 8%, less than about 9%, or less than about 10% of one or more amorphous solids and/or other crystal forms. In certain embodiments, a crystal form described herein is pure. In certain embodiments, a crystal form of a compound described herein may be about 99-99.99%, 98%, 97%, 96%, 95%, 94%, 93%, 92%, 91%, or 90% pure. The detection of other solid forms can be accomplished by, for example, diffraction analysis, thermal analysis, elemental combustion analysis and/or spectroscopic analysis. The detection of other chemical compounds can be accomplished by, for example, mass spectrometry analysis, spectroscopic analysis, thermal analysis, elemental combustion analysis and/or chromatographic analysis. As used herein, the term "form" embraces solvates and hydrates of a salt.

As used herein, the term "non-hygroscopic" refers to a crystalline salt that, after drying *in vacuo* at 50 °C for 18 hours, exhibits less than 1% weight loss between 25 °C and 200 °C upon thermogravimetric analysis at a heating rate of 20 °C/min. Also, the crystalline salt exhibits less than about 0.5% weight change upon dynamic vapor sorption analysis at 25 °C over the relative humidity (RH) range 0-90% when measured at 10% RH intervals and 60 minutes for each humidity level.

As used herein, the term "anti-solvent" refers to a solvent in which a compound or salt thereof is poorly soluble and which induces precipitation or crystallization of the compound or salt thereof from solution.

As used herein, the terms "Good Manufacturing Practices" or "GMP," "Current Good Manufacturing Practices" or "cGMP," and "Active Pharmaceutical Ingredient" or "API," follow *The International Council for Harmonisation of Technical Requirements for Pharmaceuticals for Human Use (ICH)* guidelines, which are a set of guidelines to ensure safe, effective, and high-quality medicines are developed and registered efficiently. These guidelines have been adopted by regulatory authorities throughout the world, for example, *"Q7 Good Manufacturing Practice Guidance for Active Pharmaceutical Ingredients,"* (hereinafter "Q7"), which are incorporated herein by reference.

The salts of the present disclosure can also be solvated *(i.e.,* with pharmaceutically acceptable solvents such as water, ethanol, and the like, for example, as a hydrate (e.g., monohydrate, di-hydrate, etc.)).

Broadly, an aspect of the present disclosure is directed to a crystalline salt of 2-bromolysergic acid diethylamide (2-Br-LSD), wherein the salt is a hydrobromide, hemi-fumarate, mesylate, or a besylate salt.

The structure of 2-Br-LSD is as follows: 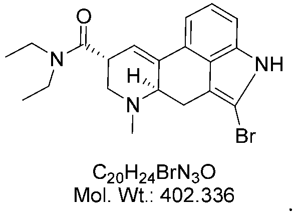 The compound 2-Br-LSD is commercially available. Alternatively, it may be prepared by known techniques. See, e.g., U.S. Patent 9,868,732.

The crystalline 2-Br-LSD salts described herein can be characterized by a number of methods including, for example, single crystal X-ray diffraction, X-ray powder diffraction (XRPD), microscopy (*e.g.,* scanning electron microscopy (SEM)), thermal analysis *(e.g.,* differential scanning calorimetry (DSC), dynamic vapor sorption (DVS), thermal gravimetric analysis (TGA), and hot-stage microscopy), spectroscopy (e.g., infrared, Raman, nuclear magnetic resonance (NMR), including ¹H, ¹³C and other nuclei in either solution or solid state), chromatography (including high performance liquid chromatography (HPLC), ultra-high performance liquid chromatography (UHPLC), ion chromatography (IC), gas chromatography (GC), and thin layer chromatography (TLC)), residue on ignition, titration, Karl Fischer moisture titration, particle size analysis, microscopy, and elemental impurities analysis by inductively coupled plasma with either optical emission spectroscopic or mass spectrometric analysis (ICP-OES or ICP-MS).

**In** some embodiments, the 2-Br-LSD crystalline salt is 2-Br-LSD hydrobromide.

In some embodiments, the 2-Br-LSD crystalline salt (2-Br-LSD hydrobromide (Form 1)) has an XRPD pattern comprising the following 2Θ peaks measured using CuK_{α} radiation: 8.0 ± 0.2, 14.2 ± 0.2, 21.9 ± 0.2, 23.3 ± 0.2, 28.6 ± 0.2, 30.4 ± 0.2. In some embodiments, the 2-Br-LSD crystalline salt (2-Br-LSD hydrobromide (Form 1)) has an XRPD pattern further comprising the following 2Θ peaks measured using CuK_{α} radiation: 12.5 ± 0.2, 17.8 ± 0.2, 19.0 ± 0.2, 24.0 ± 0.2, 24.5 ± 0.2. In some embodiments, the 2-Br-LSD crystalline salt (2-Br-LSD hydrobromide (Form 1)) has an XRPD pattern even further comprising the following 2Θ peaks measured using CuK_{α} radiation: 26.1 ± 0.2, 26.9 ± 0.2.

In some embodiments, the 2-Br-LSD crystalline salt (2-Br-LSD hydrobromide (Form 1)) has an XRPD pattern substantially similar to that set forth in FIG. 1 as measured using CuK_{α} radiation.

In some embodiments, the 2-Br-LSD crystalline salt (2-Br-LSD hydrobromide (Pattern B Solvate)) has an XRPD pattern comprising the following 2Θ peaks measured using CuK_{α} radiation: 4.4 ± 0.2, 16.9 ± 0.2. In some embodiments, the 2-Br-LSD crystalline salt (2-Br-LSD hydrobromide (Pattern B Solvate)) has an XRPD pattern futher comprising the following 2Θ peaks measured using CuK_{α} radiation: 7.7 ± 0.2, 15.0 ± 0.2, 16.1 ± 0.2, 18.1 ± 0.2, 19.5 ± 0.2, 23.0 ± 0.2, 24.7 ± 0.2, 25.5 ± 0.2.

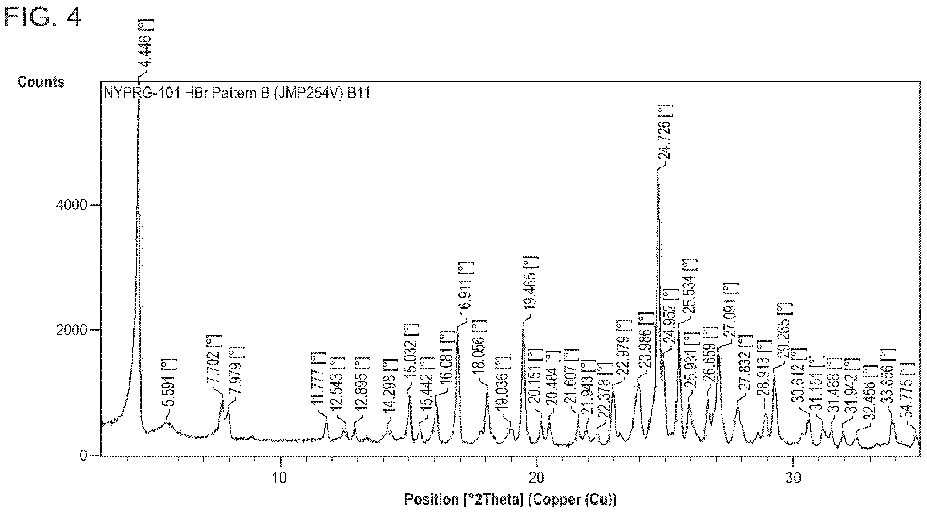

In some embodiments, the 2-Br-LSD crystalline salt (2-Br-LSD hydrobromide (Pattern B Solvate)) has an XRPD pattern substantially similar to that set forth in FIG. 4 as measured using CuKα radiation.

In some embodiments, the 2-Br-LSD crystalline salt (2-Br-LSD hydrobromide (Form 2)) has an XRPD pattern comprising the following 2Θ peaks measured using CuK_{α} radiation: 5.5 ± 0.2, 10.2 ± 0.2, 11.6 ± 0.2, 12.1 ± 0.2, 13.1 ± 0.2. In some embodiments, the 2-Br-LSD crystalline salt (2-Br-LSD hydrobromide (Form 2)) has an XRPD pattern futher comprising the following 2Θ peaks measured using CuK_{α} radiation: 7.5 ± 0.2, 13.5 ± 0.2, 16.3 ± 0.2, 18.9 ± 0.2, 19.4 ± 0.2, 20.5 ± 0.2, 21.2 ± 0.2, 24.1 ± 0.2, 24.6 ± 0.2, 24.9 ± 0.2, 25.2 ± 0.2, 25.7 ± 0.2, 26.3 ± 0.2.

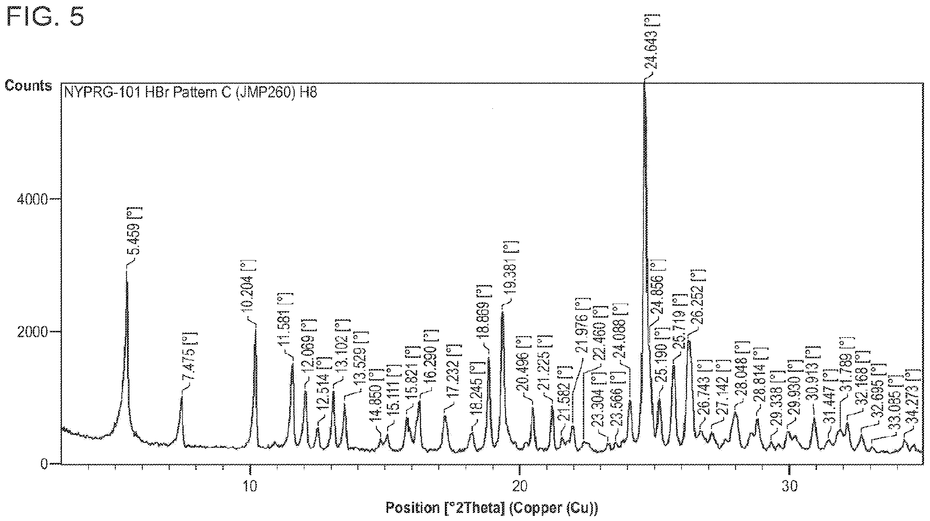

In some embodiments, the 2-Br-LSD crystalline salt (2-Br-LSD hydrobromide (Form 2)) has an XRPD pattern substantially similar to that set forth in FIG. 5 as measured using CuKα radiation.

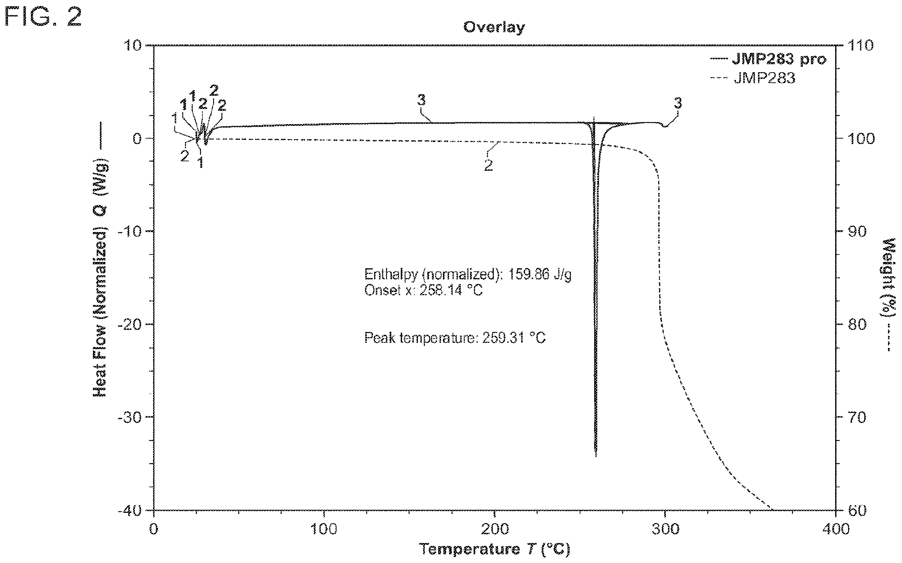

In some embodiments, the 2-Br-LSD crystalline salt (2-Br-LSD hydrobromide (Form 1)) has differential scanning calorimetry (DSC) and thermal gravimetric analysis (TGA) curves substantially similar to that set forth in FIG. 2.

In some embodiments, the 2-Br-LSD crystalline salt (2-Br-LSD hydrobromide (Form 1)) has a ¹H nuclear magnetic resonance (NMR) spectrum substantially similar to that set forth in FIG. 4.

In some embodiments, the 2-Br-LSD crystalline salt (2-Br-LSD hydrobromide (Form 1)) is non-hygroscopic.

In some embodiments, the 2-Br-LSD crystalline salt (2-Br-LSD hydrobromide (Form 1)) has an XRPD pattern comprising the following 2Θ peaks measured using CuK_{α} radiation: 8.2±0.2, 14.4±0.2, 18.0±0.2, 19.2±0.2, 20.7±0.2, 22.4±0.2, 23.4±0.2, 23.8±0.2, 24.1±0.2, 24.6±0.2, 25.4±0.2, 26.2±0.2, 27.0±0.2, 27.8±0.2, 28.1±0.2, 28.7±0.2, 30.5±0.2.

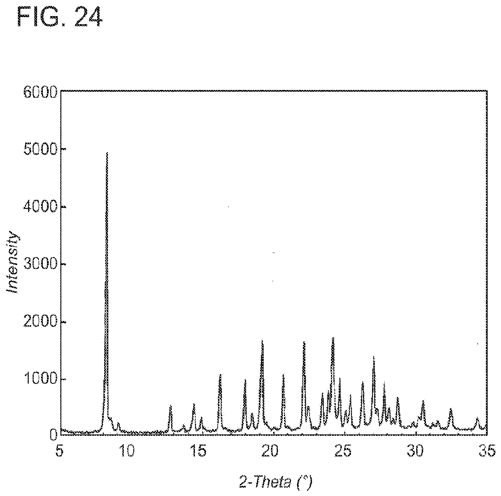

In some embodiments, the 2-Br-LSD crystalline salt (2-Br-LSD hydrobromide (Form 1)) has an XRPD pattern substantially similar to that set forth in FIG. 24 as measured using CuK_{α} radiation.

In some embodiments, the 2-Br-LSD crystalline salt (2-Br-LSD hydrobromide (Form 1)) is characterized by ¹H NMR having the following chemical shifts expressed in parts per million: 1.09 (t, 3H, J = 7 Hz, CH₃); 1.22 (t, 3H, J = 7 Hz, CH₃); 3.15 (s, 3H, N-CH₃); 2.98 - 3.78 (m, 7H, CH and CH₃); 4.40 (d, 1H, CH); 6.47 (s, 1H, CH=); 7.19 (multiplet, 3H, aromatic).

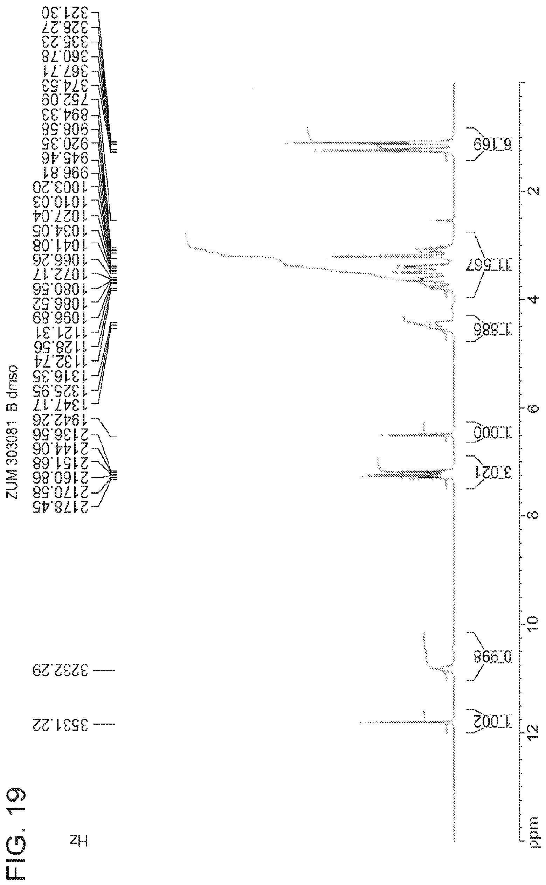

In some embodiments, the 2-Br-LSD crystalline salt (2-Br-LSD hydrobromide (Form 1)) has a ¹H NMR spectrum substantially similar to that set forth in FIG. 19.

In some embodiments, the 2-Br-LSD crystalline salt (2-Br-LSD hydrobromide (Form 1)) is characterized by ¹³C NMR having the following chemical shifts expressed in parts per million: 13.0 (CH₃); 14.7 (CH₃); 23.2; 36.4; 40.0 (CH₃); 41.1; 41.9; 53.3 (CH₂-N); 60.9 (CH-N); 104.8; 106.5; 110.4; 112.8; 120.2 (CH=); 123.1; 124.0; 125.3; 130.7; 134.1; 167.9 (C=O).

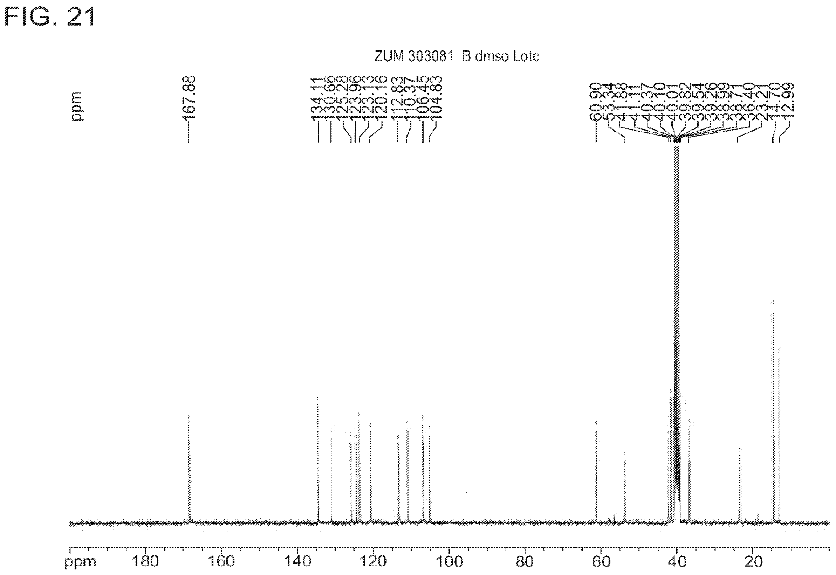

In some embodiments, the 2-Br-LSD crystalline salt (2-Br-LSD hydrobromide (Form 1)) has a ¹³C NMR spectrum substantially similar to that set forth in FIG. 21.

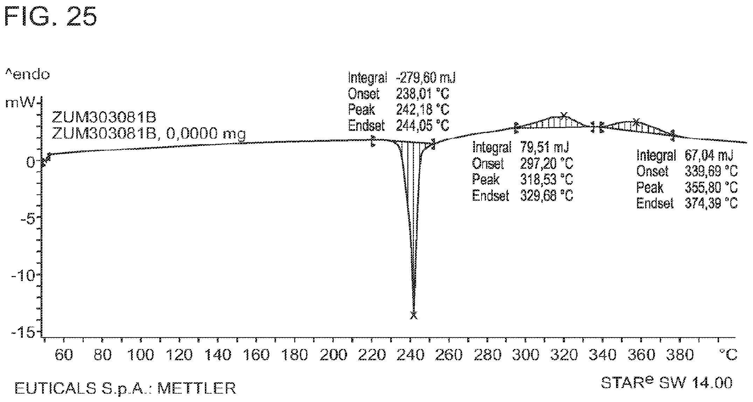

In some embodiments, the 2-Br-LSD crystalline salt (2-Br-LSD hydrobromide (Form 1)) has a DSC curve substantially similar to that set forth in FIG. 25.

In some embodiments, the 2-Br-LSD crystalline salt (2-Br-LSD hydrobromide (Form 1)) is made according to GMP (also referred to as "Example 2, Method 4").

**In** some embodiments, the 2-Br-LSD crystalline salt is 2-Br-LSD hemi-fumarate.

In some embodiments, the 2-Br-LSD crystalline salt (2-Br-LSD hemi-fumarate (Form A)) salt has an XRPD pattern comprising the following 2Θ peaks measured using CuK_{α} radiation: 4.4 ± 0.2, 7.4 ± 0.2, 8.3 ± 0.2, 10.3 ± 0.2, 14.9 ± 0.2, 21.9 ± 0.2. In some embodiments, the 2-Br-LSD crystalline salt (2-Br-LSD hemi-fumarate (Form A)) has an XRPD pattern further comprising the following 2Θ peaks measured using CuKα radiation: 9.5 ± 0.2, 11.5 ± 0.2, 11.9 ± 0.2, 13.5 ± 0.2, 16.3 ± 0.2, 17.1 ± 0.2, 18.3 ± 0.2, 19.3 ± 0.2, 19.8 ± 0.2, 20.5 ± 0.2, 20.8 ± 0.2, 21.5 ± 0.2, 22.5 ± 0.2, 23.2 ± 0.2, 23.8 ± 0.2, 25.1 ± 0.2, 26.8 ± 0.2, 28.7 ± 0.2, 30.1 ± 0.2, 30.6 ± 0.2.

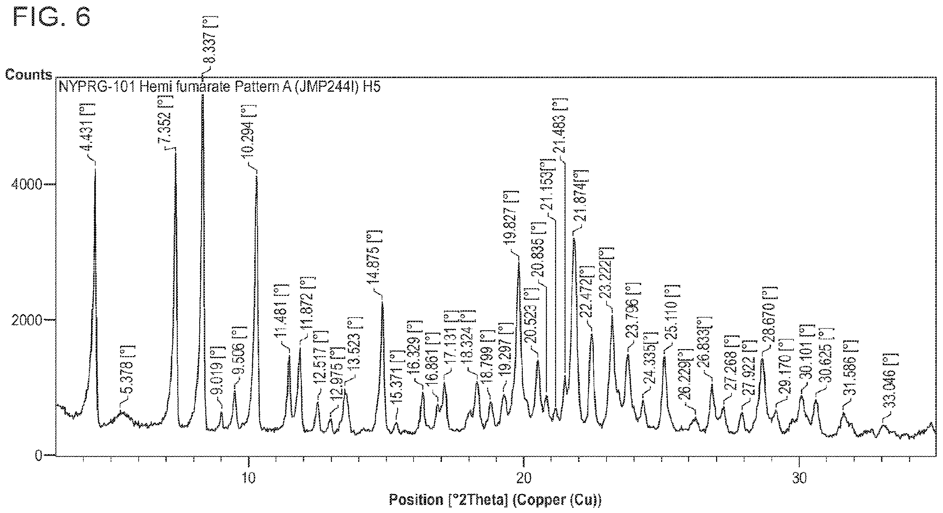

In some embodiments, the 2-Br-LSD crystalline salt (2-Br-LSD hemi-fumarate (Form A)) has an XRPD pattern substantially similar to that set forth in FIG. 6. as measured using CuKα radiation.

In some embodiments, the 2-Br-LSD crystalline salt is 2-Br-LSD mesylate.

In some embodiments, the 2-Br-LSD crystalline salt (2-Br-LSD mesylate (Form B)) has an XRPD pattern comprising the following 2Θ peaks measured using CuK_{α} radiation: 7.5 ± 0.2. In some embodiments, the 2-Br-LSD crystalline salt (2-Br-LSD mesylate (Form B)) has an XRPD pattern further comprising the following 2Θ peaks measured using CuK_{α} radiation: 7.2 ± 0.2, 10.1 ± 0.2, 10.8 ± 0.2, 13.3 ± 0.2, 15.1 ± 0.2, 15.5 ± 0.2, 17.2 ± 0.2, 19.4 ± 0.2, 20.5 ± 0.2, 21.6 ± 0.2, 22.3 ± 0.2, 23.2 ± 0.2, 23.5 ± 0.2, 24.4 ± 0.2, 26.1 ± 0.2, 28.1 ± 0.2.

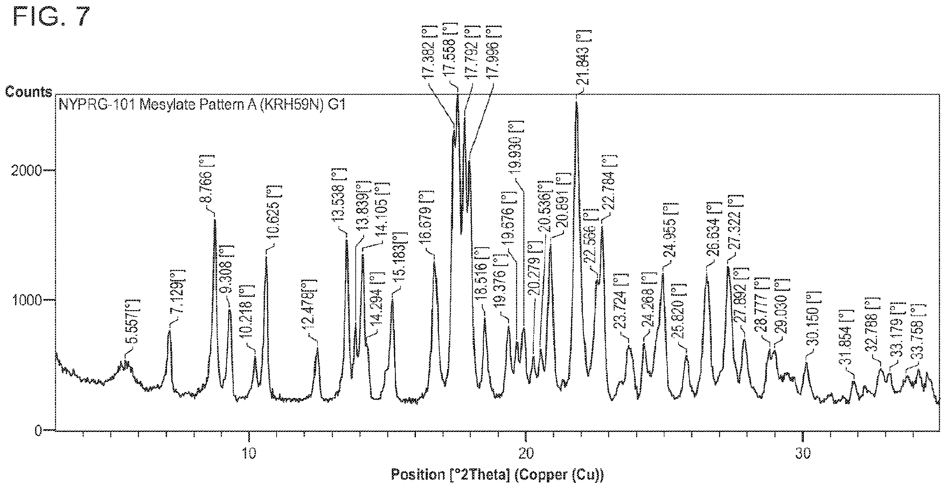

In some embodiments, the 2-Br-LSD crystalline salt (2-Br-LSD mesylate (Form B)) has an XRPD pattern substantially similar to that set forth in FIG. 7 as measured using CuKα radiation.

In some embodiments, the 2-Br-LSD crystalline salt is 2-Br-LSD besylate.

In some embodiments, the 2-Br-LSD crystalline salt (2-Br-LSD besylate) has an XRPD pattern comprising the following 2Θ peaks measured using CuK_{α} radiation: 5.9 ± 0.2, 8.7 ± 0.2, 17.8 ± 0.2, 19.7 ± 0.2, 23.4 ± 0.2. In some embodiments, the 2-Br-LSD crystalline salt (2-Br-LSD besylate) has an XRPD pattern further comprising the following 2Θ peaks measured using CuK_{α} radiation: 13.4 ± 0.2, 13.8 ± 0.2, 14.6 ± 0.2, 16.1 ± 0.2, 16.3 ± 0.2, 16.8 ± 0.2, 20.5 ± 0.2, 21.6 ± 0.2, 22.3 ± 0.2, 22.9 ± 0.2, 23.8 ± 0.2, 24.5 ± 0.2.

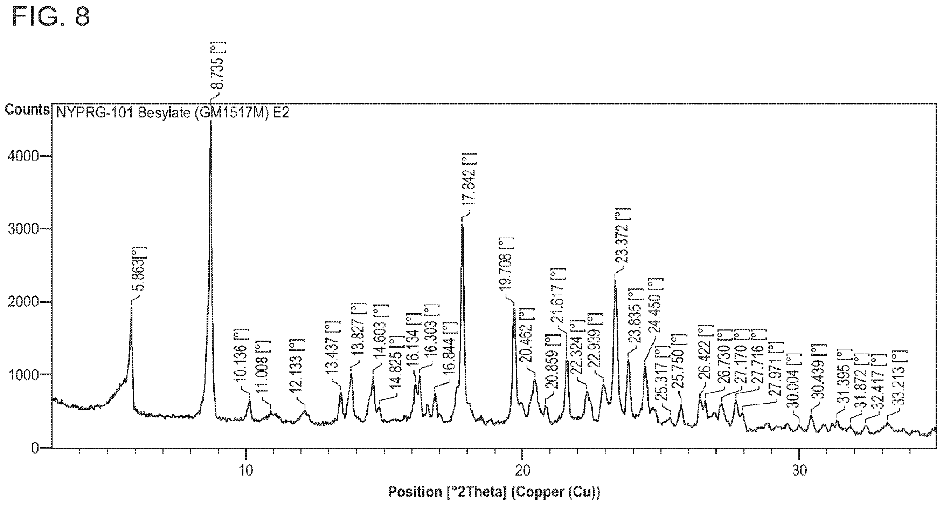

In some embodiments, the crystalline salt (2-Br-LSD besylate) has an XRPD pattern substantially similar to that set forth in FIG. 8 as measured using CuKα radiation.

The crystalline salts of 2-Br-LSD were characterized by their powder X-ray diffraction patterns. Powder X-ray diffractograms of the crystalline salts of 2-Br-LSD described herein were collected on a PANalytical X'pert PRO diffractometer using Cu Kα radiation (45kV, 40mA), θ - θ goniometer, focusing mirror, divergence slit (1/2"), soller slits at both incident and divergent beam (4mm) and a PIXcel detector. Data was presented using X'Pert Data Viewer, version 1.2d. XRPD patterns were acquired under ambient conditions via a transmission foil sample stage (polyimide - Kapton^{®}, 12.7µm thickness film). The data collection range was 2.994 - 35°2θ with a continuous scan speed of 0.202004°s⁻¹. Lists of major XRPD peaks (relative intensity ≥10%) for the crystalline salts of the present disclosure are tabulated below.

An additional XRPD pattern for 2-Br-LSD hydrobromide (Form 1) was collected using a Bruker D2 X-ray Generator (30 kV, 10 mA, scan interval 3-35°, Step 0.04°, 2 sec/step; flat plate zero-background sample holder, rotation 15 rpm). See, FIG. 24 and Table 2D.

A variety of factors, including instrument design, maintenance, calibration and mode of operation, specimen preparation and presentation, particle size and inhomogeneity, preferred orientation, and environmental conditions can cause variability in the measurement of diffraction angles and peak intensities. Angle measurements are presented herein as ±0.2° 2θ. Relative intensities can vary widely. As used in the description and the appended claims, the phrase "an XRPD pattern substantially similar to" a reference pattern takes into account these sources of experimental variability. For example, the XRPD patterns for 2-Br-LSD hydrobromide (Form 1) presented in FIG. 1 (with peaks listed in Table 2A) and FIG. 24 (with peaks listed in Table 2D) are "substantially similar" as defined herein, even though the corresponding 2θ values are offset by nearly 0.2° and the relative intensities for certain peaks (e.g., ~8.0° and ~24.0° 2θ) are very different. These differences in relative intensities may be due to "preferred orientation" in one or both of the specimens, as is well understood in the art.

The following tables list the 2θ and intensities of lines for the crystalline salts of 2-Br-LSD. In cases where there are two or more crystalline forms of a 2-Br-LSD salt, each form can be identified and distinguished from the other crystalline form by either a single x-ray diffraction line, a combination of lines, or a pattern that is different from the x-ray powder diffraction of the other forms. Some diffraction lines that are unique to a given solid form with respect to the other known forms of the same salt are indicated in **boldface** type.

XRPD diffraction peaks for crystalline 2-Br-LSD mesylate Forms A, B, and C are listed in Tables 1A-1C, respectively.

**Table 1A. 2-Br-LSD mesylate (Form A), PANalytical diffractometer**

| Pos. [°2Th.] | Rel. Int. [%] |
|---|---|
| 7.1 ± 0.2 | 20.6 |
| **8.8 ± 0.2** | 59.4 |
| 9.3 ± 0.2 | 28.3 |
| 10.6 ± 0.2 | 46.7 |
| 12.5 ± 0.2 | 16.8 |
| 13.5 ± 0.2 | 52.9 |
| 13.8 ± 0.2 | 25.0 |
| 14.1 ± 0.2 | 48.4 |
| 15.2 ± 0.2 | 33.1 |
| **16.7 ± 0.2** | 44.7 |
| 17.4 ± 0.2 | 85.0 |
| **17.6 ± 0.2** | 100.0 |
| **17.8 ± 0.2** | 94.1 |
| **18.0 ± 0.2** | 78.7 |
| 18.5 ± 0.2 | 27.9 |
| 19.4 ± 0.2 | 25.0 |
| 19.7 ± 0.2 | 19.8 |
| 19.9 ± 0.2 | 24.3 |
| 20.5 ± 0.2 | 17.5 |
| 20.9 ± 0.2 | 51.8 |
| 21.8 ± 0.2 | 99.2 |
| 22.6 ± 0.2 | 40.4 |
| 22.8 ± 0.2 | 56.5 |
| 23.7 ± 0.2 | 18.7 |
| 24.3 ± 0.2 | 19.4 |
| **25.0 ± 0.2** | 42.2 |
| **26.6 ± 0.2** | 33.6 |
| **27.3 ± 0.2** | 45.1 |
| 27.9 ± 0.2 | 20.8 |

**Table 1B. 2-Br-LSD mesylate (Form B). PANalytical diffractometer**

| Pos. [°2Th.] | Rel. Int. [%] |
|---|---|
| 7.2 ± 0.2 | 28.0 |
| **7.5 ± 0.2** | 19.6 |
| 10.1 ± 0.2 | 10.8 |
| 10.8 ± 0.2 | 13.3 |
| 13.3 ± 0.2 | 43.0 |
| 15.1 ± 0.2 | 18.3 |
| 15.5 ± 0.2 | 30.4 |
| 17.2 ± 0.2 | 11.8 |
| 19.4 ± 0.2 | 100.0 |
| 20.5 ± 0.2 | 31.0 |
| 21.6 ± 0.2 | 30.6 |
| 22.3 ± 0.2 | 23.4 |
| 23.2 ± 0.2 | 16.5 |
| 23.5 ± 0.2 | 15.1 |
| 24.4 ± 0.2 | 23.5 |
| 26.1 ± 0.2 | 11.5 |
| 28.1 ± 0.2 | 12.2 |

**Table 1C. 2-Br-LSD mesylate (Form C). PANalytical diffractometer**

| Pos. [°2Th.] | Rel. Int. [%] |
|---|---|
| **3.3 ± 0.2** | 30.3 |
| **5.0 ± 0.2** | 100.0 |
| **5.4 ± 0.2** | 78.9 |
| **6.6 ± 0.2** | 46.5 |
| 7.2 ± 0.2 | 12.2 |
| 10.1 ± 0.2 | 11.0 |
| 10.8 ± 0.2 | 14.8 |
| 12.2 ± 0.2 | 15.2 |
| 13.3 ± 0.2 | 18.9 |
| 14.3 ± 0.2 | 13.4 |
| 15.0 ± 0.2 | 17.3 |
| 15.5 ± 0.2 | 16.3 |
| 18.7 ± 0.2 | 26.5 |
| 19.4 ± 0.2 | 49.6 |
| 20.5 ± 0.2 | 22.8 |
| 21.6 ± 0.2 | 26.5 |
| 22.3 ± 0.2 | 21.0 |
| 23.2 ± 0.2 | 30.9 |
| 24.4 ± 0.2 | 37.0 |

[0072]XRPD diffraction peaks for crystalline 2-Br-LSD hydrobromide (Form 1), Pattern B Solvate, and Form 2 are listed in Tables 2A-2D.

**Table 2A. 2-Br-LSD hydrobromide (Form 1), PANalytical diffractometer**

| Pos. [°2Th.] | Rel. Int. [%] |
|---|---|
| **8.0 ± 0.2** | 83.9 |
| 12.5 ± 0.2 | 28.8 |
| **14.2 ± 0.2** | 25.0 |
| 14.7 ± 0.2 | 10.5 |
| 16.0 ± 0.2 | 16.8 |
| 17.8 ± 0.2 | 17.6 |
| 18.3 ± 0.2 | 10.7 |
| 18.9 ± 0.2 | 15.8 |
| 19.0 ± 0.2 | 30.4 |
| 20.5 ± 0.2 | 18.0 |
| **21.9 ± 0.2** | 28.7 |
| 22.3 ± 0.2 | 10.1 |
| **23.3 ± 0.2** | 17.8 |
| 23.7 ± 0.2 | 18.1 |
| 24.0 ± 0.2 | 100.0 |
| 24.5 ± 0.2 | 55.3 |
| 24.9 ± 0.2 | 10.5 |
| 25.2 ± 0.2 | 15.3 |
| 26.1 ± 0.2 | 24.1 |
| 26.9 ± 0.2 | 25.6 |
| 27.1 ± 0.2 | 14.9 |
| 27.7 ± 0.2 | 15.4 |
| 28.0 ± 0.2 | 16.8 |
| **28.6 ± 0.2** | 19.9 |
| **30.4 ± 0.2** | 19.5 |

**Table 2B. 2-Br-LSD hydrobromide (Pattern B Solvate), PANalytical diffractometer**

| Pos. [°2Th.] | Rel. Int. [%] |
|---|---|
| **4.4 ± 0.2** | 100.0 |
| 7.7 ± 0.2 | 10.9 |
| 15.0 ± 0.2 | 13.5 |
| 16.1 ± 0.2 | 11.3 |
| **16.9 ± 0.2** | 31.8 |
| 18.1 ± 0.2 | 14.7 |
| 19.5 ± 0.2 | 33.0 |
| 23.0 ± 0.2 | 15.0 |
| 24.0 ± 0.2 | 15.7 |
| 24.7 ± 0.2 | 78.0 |
| 25.0 ± 0.2 | 22.1 |
| 25.5 ± 0.2 | 33.4 |
| 25.9 ± 0.2 | 11.7 |
| 26.7 ± 0.2 | 12.9 |
| 27.1 ± 0.2 | 25.8 |
| 27.8 ± 0.2 | 10.8 |
| 29.3 ± 0.2 | 19.8 |

**Table 2C. 2-Br-LSD hydrobromide (Form 2), PANalytical diffractometer**

| Pos. [°2Th.] | Rel. Int. [%] |
|---|---|
| **5.5 ± 0.2** | 45.9 |
| 7.5 ± 0.2 | 13.2 |
| **10.2 ± 0.2** | 32.2 |
| **11.6 ± 0.2** | 23.7 |
| **12.1 ± 0.2** | 16.3 |
| **13.1 ± 0.2** | 15.8 |
| 13.5 ± 0.2 | 12.3 |
| 16.3 ± 0.2 | 13.4 |
| 18.9 ± 0.2 | 25.1 |
| 19.4 ± 0.2 | 37.8 |
| 20.5 ± 0.2 | 11.9 |
| 21.2 ± 0.2 | 12.0 |
| 24.1 ± 0.2 | 13.5 |
| 24.6 ± 0.2 | 100.0 |
| 24.9 ± 0.2 | 23.1 |
| 25.2 ± 0.2 | 14.2 |
| 25.7 ± 0.2 | 23.3 |
| 26.3 ± 0.2 | 29.0 |

**Table 2D. 2-Br-LSD hydrobromide (Form 1), Bruker D2 X-ray Generator**

| Pos. [°2Th.] | Rel. Int. [%] |
|---|---|
| 8.2 ± 0.2 | 100.0 |
| 12.7 ± 0.2 | 10.9 |
| 14.4 ± 0.2 | 11.4 |
| 16.2 ± 0.2 | 21.7 |
| 18.0 ± 0.2 | 19.4 |
| 19.2 ± 0.2 | 33.8 |
| 20.7 ± 0.2 | 22.1 |
| 22.1 ± 0.2 | 33.1 |
| 22.4 ± 0.2 | 10.6 |
| 23.4 ± 0.2 | 15.3 |
| 23.8 ± 0.2 | 15.9 |
| 24.1 ± 0.2 | 34.7 |
| 24.6 ± 0.2 | 20.6 |
| 25.4 ± 0.2 | 14.7 |
| 26.2 ± 0.2 | 19.3 |
| 27.0 ± 0.2 | 28.2 |
| 27.8 ± 0.2 | 19.1 |
| 28.1 ± 0.2 | 10.2 |
| 28.7 ± 0.2 | 13.7 |
| 30.5 ± 0.2 | 12.6 |

XRPD diffraction peaks for crystalline 2-Br-LSD xinafoate Forms A and B are listed in Tables 3A-3B.

**Table 3A. 2-Br-LSD xinafoate (Form A), PANalytical diffractometer**

| Pos. [°2Th.] | Rel. Int. [%] |
|---|---|
| 5.5 ± 0.2 | 78.9 |
| 6.5 ± 0.2 | 71.5 |
| 8.9 ± 0.2 | 66.7 |
| 10.6 ± 0.2 | 67.5 |
| 11.2 ± 0.2 | 35.1 |
| 13.1 ± 0.2 | 86.3 |
| 14.9 ± 0.2 | 35.9 |
| 16.0 ± 0.2 | 87.0 |
| 16.5 ± 0.2 | 62.7 |
| 17.1 ± 0.2 | 51.3 |
| 17.6 ± 0.2 | 66.7 |
| 17.9 ± 0.2 | 43.4 |
| 18.8 ± 0.2 | 71.0 |
| 19.4 ± 0.2 | 50.7 |
| 20.1 ± 0.2 | 41.5 |
| 20.7 ± 0.2 | 13.8 |
| 21.4 ± 0.2 | 25.2 |
| 22.0 ± 0.2 | 54.7 |
| 22.4 ± 0.2 | 100.0 |
| 23.1 ± 0.2 | 21.1 |
| 24.4 ± 0.2 | 12.8 |
| 25.5 ± 0.2 | 36.0 |
| 27.4 ± 0.2 | 24.2 |

**Table 3B. 2-Br-LSD xinafoate (Form B), PANalytical diffractometer**

| Pos. [°2Th.] | Rel. Int. [%] |
|---|---|
| 6.4 ± 0.2 | 71.7 |
| 8.6 ± 0.2 | 37.2 |
| 8.9 ± 0.2 | 59.1 |
| 9.2 ± 0.2 | 25.9 |
| 10.5 ± 0.2 | 67.8 |
| 11.1 ± 0.2 | 32.8 |
| 13.1 ± 0.2 | 80.4 |
| 13.3 ± 0.2 | 25.0 |
| 14.8 ± 0.2 | 36.0 |
| 15.4 ± 0.2 | 25.1 |
| 15.8 ± 0.2 | 79.3 |
| 16.4 ± 0.2 | 64.4 |
| 16.7 ± 0.2 | 20.2 |
| 17.0 ± 0.2 | 50.0 |
| 17.5 ± 0.2 | 67.3 |
| 17.8 ± 0.2 | 49.0 |
| 18.2 ± 0.2 | 43.8 |
| 18.5 ± 0.2 | 45.0 |
| 18.7 ± 0.2 | 68.0 |
| 19.4 ± 0.2 | 54.3 |
| 20.0 ± 0.2 | 46.1 |
| 20.7 ± 0.2 | 24.7 |
| 21.3 ± 0.2 | 32.8 |
| 21.9 ± 0.2 | 62.8 |
| 22.3 ± 0.2 | 100.0 |
| 23.0 ± 0.2 | 30.0 |
| 25.4 ± 0.2 | 43.0 |
| 26.1 ± 0.2 | 19.6 |
| 27.3 ± 0.2 | 31.5 |

XRPD diffraction peaks for crystalline 2-Br-LSD oxalate Forms A and B are listed in Tables 4A-4B.

**Table 4A. 2-Br-LSD oxalate (Form A), PANalytical diffractometer**

| Pos. [°2Th.] | Rel. Int. [%] |
|---|---|
| 3.7 ± 0.2 | 11.9 |
| 5.1 ± 0.2 | 42.0 |
| 6.3 ± 0.2 | 13.7 |
| **7.8 ± 0.2** | 21.3 |
| **8.3 ± 0.2** | 100.0 |
| 12.7 ± 0.2 | 13.0 |
| 17.1 ± 0.2 | 10.6 |
| 19.5 ± 0.2 | 25.1 |
| 22.6 ± 0.2 | 31.3 |
| 23.1 ± 0.2 | 22.1 |
| 23.5 ± 0.2 | 23.1 |
| 23.8 ± 0.2 | 43.0 |
| 24.3 ± 0.2 | 17.4 |
| 24.6 ± 0.2 | 24.8 |
| 25.5 ± 0.2 | 16.9 |
| 25.9 ± 0.2 | 31.4 |
| 26.7 ± 0.2 | 11.1 |

**Table 4B. 2-Br-LSD oxalate (Form B), PANalytical diffractometer**

| Pos. [°2Th.] | Rel. Int. [%] |
|---|---|
| 4.0 ± 0.2 | 32.2 |
| 5.8 ± 0.2 | 76.6 |
| 7.1 ± 0.2 | 25.5 |
| **8.7 ± 0.2** | 60.6 |
| **9.2 ± 0.2** | 48.0 |
| **9.8 ± 0.2** | 69.1 |
| 11.1 ± 0.2 | 20.3 |
| **12.0** ± **0.2** | 60.1 |
| **12.3** ± **0.2** | 85.3 |
| 13.3 ± 0.2 | 18.0 |
| 14.3 ± 0.2 | 19.0 |
| **14.9 ± 0.2** | 30.7 |
| 16.8 ± 0.2 | 50.8 |
| 17.7 ± 0.2 | 25.2 |
| 18.4 ± 0.2 | 26.8 |
| 18.6 ± 0.2 | 26.3 |
| 19.1 ± 0.2 | 42.7 |
| 19.5 ± 0.2 | 39.0 |
| 19.8 ± 0.2 | 51.8 |
| 20.6 **±** 0.2 | 21.9 |
| **21.5** ± **0.2** | 48.8 |
| 22.6 ± 0.2 | 41.3 |
| 23.3 ± 0.2 | 100.0 |
| 24.3 ± 0.2 | 64.8 |
| 24.7 ± 0.2 | 38.1 |
| 25.9 ± 0.2 | 21.7 |
| 27.1 ± 0.2 | 23.4 |
| 27.7 ± 0.2 | 16.7 |

XRPD diffraction peaks for crystalline 2-Br-LSD hemi-fumarate Forms A and B are listed in Tables 5A-5B.

**Table 5A. 2-Br-LSD hemi-fumarate (Form A), PANalytical diffractometer**

| Pos. [°2Th.] | Rel. Int. [%] |
|---|---|
| **4.4 ± 0.2** | 73.08 |
| **7.4 ± 0.2** | 78.0 |
| **8.3 ± 0.2** | 100.0 |
| 9.5 ± 0.2 | 10.4 |
| **10.3 ± 0.2** | 72.1 |
| 11.5 ± 0.2 | 21.3 |
| 11.9 ± 0.2 | 23.5 |
| 13.5 ± 0.2 | 11.7 |
| **14.9 ± 0.2** | 36.8 |
| 16.3 ± 0.2 | 11.5 |
| 17.1 ± 0.2 | 14.2 |
| 18.3 ± 0.2 | 14.5 |
| 19.3 ± 0.2 | 10.9 |
| 19.8 ± 0.2 | 48.8 |
| 20.5 ± 0.2 | 20.9 |
| 20.8 ± 0.2 | 10.8 |
| 21.5 ± 0.2 | 16.0 |
| **21.9 ± 0.2** | 50.9 |
| 22.5 ± 0.2 | 28.6 |
| 23.2 ± 0.2 | 33.4 |
| 23.8 ± 0.2 | 22.8 |
| 25.1 ± 0.2 | 22.2 |
| 26.8 ± 0.2 | 13.4 |
| 28.7 ± 0.2 | 21.5 |
| 30.1 ± 0.2 | 11.0 |
| 30.6 ± 0.2 | 10.0 |

**Table 5B. 2-Br-LSD hemi-fumarate (Form B), PANalytical diffractometer**

| Pos. [°2Th.] | Rel. Int. [%] |
|---|---|
| **7.0 ± 0.2** | 23.5 |
| **8.8 ± 0.2** | 100.0 |
| 12.5 ± 0.2 | 31.0 |
| 15.4 ± 0.2 | 18.3 |
| 17.1 ± 0.2 | 10.3 |
| 18.7 ± 0.2 | 11.1 |
| 20.0 ± 0.2 | 25.4 |
| 20.7 ± 0.2 | 41.3 |
| 22.6 ± 0.2 | 20.9 |
| 24.5 ± 0.2 | 11.2 |
| 28.6 ± 0.2 | 13.1 |

XRPD diffraction peaks for crystalline 2-Br-LSD hemi-succinate Form A are listed in Table 6.

**Table 6. 2-Br-LSD hemi-succinate Form A, PANalytical diffractometer**

| Pos. [°2Th.] | Rel. Int. [%] |
|---|---|
| 5.4 ± 0.2 | 12.3 |
| 6.6 ± 0.2 | 100.0 |
| 8.7 ± 0.2 | 28.9 |
| 11.0 ± 0.2 | 13.4 |
| 12.0 ± 0.2 | 11.6 |
| 18.0 ± 0.2 | 11.8 |
| 19.1 ± 0.2 | 14.6 |
| 19.9 ± 0.2 | 24.9 |
| 20.9 ± 0.2 | 25.4 |
| 21.9 ± 0.2 | 35.9 |
| 24.9 ± 0.2 | 11.0 |
| 26.0 ± 0.2 | 21.8 |

XRPD diffraction peaks for crystalline 2-Br-LSD besylate are listed in Table 7.

**Table 7. 2-Br-LSD besylate, PANalytical diffractometer**

| Pos. [°2Th.] | Rel. Int. [%] |
|---|---|
| 5.9 ± 0.2 | 33.4 |
| 8.7 ± 0.2 | 100.0 |
| 13.4 ± 0.2 | 10.1 |
| 13.8 ± 0.2 | 15.6 |
| 14.6 ± 0.2 | 15.2 |
| 16.1 ± 0.2 | 12.8 |
| 16.3 ± 0.2 | 15.1 |
| 16.8 ± 0.2 | 10.0 |
| 17.8 ± 0.2 | 64.5 |
| 19.7 ± 0.2 | 37.9 |
| 20.5 ± 0.2 | 14.9 |
| 21.6 ± 0.2 | 21.0 |
| 22.3 ± 0.2 | 11.4 |
| 22.9 ± 0.2 | 13.5 |
| 23.4 ± 0.2 | 46.7 |
| 23.8 ± 0.2 | 21.6 |
| 24.5 ± 0.2 | 19.8 |

XRPD diffraction peaks for crystalline mono-succinate (Form A) are listed in Table 8.

**Table 8. 2-Br-LSD mono-succinate (Form A), PANalytical diffractometer**

| Pos. [°2Th.] | Rel. Int. [%] |
|---|---|
| 5.4 ± 0.2 | 12.3 |
| 6.6 ± 0.2 | 100.0 |
| 8.7 ± 0.2 | 28.9 |
| 11.0 ± 0.2 | 13.4 |
| 12.0 ± 0.2 | 11.6 |
| 15.1 ± 0.2 | 9.1 |
| 18.0 ± 0.2 | 11.8 |
| 19.1 ± 0.2 | 14.6 |
| 19.9 ± 0.2 | 24.9 |
| 20.9 ± 0.2 | 25.4 |
| 21.9 ± 0.2 | 35.9 |
| 24.9 ± 0.2 | 11.0 |
| 26.0 ± 0.2 | 21.8 |
| 27.0 ± 0.2 | 8.5 |
| 29.3 ± 0.2 | 9.9 |
| 31.4 ± 0.2 | 7.7 |
| 32.3 ± 0.2 | 6.9 |

### Methods of Preparation

In another aspect, a method of preparing the crystalline salt of 2-Br-LSD hydrobromide (Form 1), which has an XRPD pattern substantially similar to that set forth in FIG. 1 as measured using CuK_{α} radiation, comprises the following steps:
(a) equilibrating a solution comprising 2-Br-LSD and a solvent at a first temperature;
(b) adding a first portion of hydrobromic acid to the first-temperature equilibrated solution;
(c) equilibrating the batch at the first temperature for a period of time M;
(d) adding a second portion of hydrobromic acid to the batch over a period of time N;
(e) equilibrating the batch at the first temperature for a period of time O;
(f) cooling the batch to a second temperature;
(g) equilibrating the batch at the second temperature for a period of time P;
(h) isolating precipitate from the suspension; and
(i) drying the isolated precipitate *in vacuo* at a third temperature to obtain the crystalline salt;
   wherein M is between 0 and 24 hours;
   wherein N is between 5 minutes and 24 hours;
   wherein O is between 0 and 8 hours;
   wherein P is between 30 minutes and 48 hours.

In some embodiments, the above method further comprises adding seed crystals of 2-Br-LSD hydrobromide (Form 1) to the batch prior to step (c).

In some embodiments, the solvent comprises water and ethanol, methanol or isopropanol.

In some embodiments, the solvent comprises 2-12% water. In some embodiments, the solvent comprises ethanol. In some embodiments, the solvent comprises 88% to 98% ethanol

In some embodiments, the solvent is ethanol-water in a ratio of about 95:5 v/v.

In some embodiments, the concentration of 2-Br-LSD in step (a) is between 0.12M and 0.6M.

In some embodiments, the concentration of 2-Br-LSD in step (a) is between 0.2M and 0.4M.

In some embodiments, the hydrobromic acid is added as a solution.

In some embodiments, the hydrobromic acid is added as a solution in the same solvent used in step (a).

In some embodiments, the hydrobromic acid is added as a solution in ethanol-water at a concentration of 0.5M to 4M.

In some embodiments, the hydrobromic acid is added as a solution in the same solvent used in step (a) at a concentration of 1M to 3M.

In some embodiments, the first temperature is between about 20 °C and 50 °C.

In some embodiments, the first temperature is between 30 °C and 50 °C and the second temperature is between 0 °C and about 25 °C.

In some embodiments, the first temperature is between 40 °C and 50 °C and the second temperature is about 20 °C to about 25 °C.

In some embodiments, M is between 1 and 4 hours, N is between 2 and 6 hours, O is between 30 minutes and 4 hours, and P is between 8 and 24 hours.

In some embodiments, the method further comprises:
following period of time P and prior to step (h), cooling the batch to a fourth temperature and equilibrating the batch at the fourth temperature for a period of time Q;
wherein Q is between 5 minutes and 48 hours.

In some embodiments the fourth temperature is between 0 °C and 10 °C.

In some embodiments, Q is between 0.5 hour and 4 hours.

In some embodiments, the first temperature is about 20 °C to about 25 °C and the second temperature is between 0 °C and 10 °C.

In some embodiments, M is between 1 and 4 hours, N is between 2 and 6 hours, O is between 30 minutes and 4 hours, and P is between 8 and 24 hours.

In some embodiments, the sum of the hydrobromic acid portions in steps (b) and (d) is 1.0 molar equivalent with respect to 2-Br-LSD.

In some embodiments, the portion of hydrobromic acid in step (b) is sufficient to induce the formation of crystals of 2-Br-LSD hydrobromide.

In some embodiments, the addition of hydrobromic acid in step (b) is halted at the first visible sign of crystallization.

In some embodiments, the method further comprises adding seed crystals of 2-Br-LSD hydrobromide (Form 1) to the batch prior to step (c).

In some embodiments, the hydrobromic acid is added as a gas in steps (b) and (d).

In some embodiments, M is 0.

In some embodiments, the hydrobromic acid portion in step (b) is between 0.1 and 0.4 molar equivalents with respect to 2-Br-LSD.

In some embodiments, the hydrobromic acid portion in step (b) is between 0.2 and 0.3 molar equivalents with respect to 2-Br-LSD.

Representative methods of preparing the other 2-Br-LSD salts disclosed herein are described in various non-limiting working examples.

### Pharmaceutical Compositions

Another aspect of the present disclosure is directed to a pharmaceutical composition that comprises a therapeutically effective amount of a 2-Br-LSD salt of the present disclosure and a pharmaceutically acceptable carrier. The term "pharmaceutically acceptable carrier," as known in the art, refers to a pharmaceutically acceptable material, composition or vehicle, suitable for administering a crystalline 2-Br-LSD salt of the present disclosure to mammals. Suitable carriers may include, for example, liquids (both aqueous and non-aqueous alike, and combinations thereof), solids, encapsulating materials, and combinations thereof (e.g., semi-solids). A carrier is "pharmaceutically acceptable" in the sense of being physiologically inert, compatible with the other ingredients of the formulation, and not injurious to the subject or patient. Depending on the type of formulation, the composition may further include one or more pharmaceutically acceptable excipients.

Broadly, 2-Br-LSD salts of the present disclosure may be formulated into a given type of composition in accordance with conventional pharmaceutical practice such as conventional mixing, dissolving, granulating, dragee-making, levigating, emulsifying, encapsulating, entrapping and compression processes (*see, e.g.,* Remington: The Science and Practice of Pharmacy (20th ed.), ed. A. R. Gennaro, Lippincott Williams & Wilkins, 2000 *and* Encyclopedia of Pharmaceutical Technology, eds. J. Swarbrick and J. C. Boylan, 1988-1999, Marcel Dekker, New York).

In some embodiments, the 2-Br-LSD salts are formulated for oral or intravenous administration (e.g., systemic intravenous injection).

The 2-Br-LSD salts of the present disclosure may be formulated into solid compositions (*e.g*., powders, tablets, dispersible granules, capsules, cachets, and suppositories), liquid compositions (*e.g.*, solutions in which the 2-Br-LSD salt is dissolved, suspensions in which solid particles of the 2-Br-LSD salt are dispersed, emulsions, and solutions containing liposomes, micelles, or nanoparticles, syrups and elixirs). The 2-Br-LSD salts may also be formulated for rapid, intermediate or extended release.

In some embodiments, the pharmaceutical composition is in the form of a solid or a liquid.

As shown in Example 2 (Method 4), the 2-Br-LSD salt, e.g., a hydrobromide salt of 2-Br-LSD having the solid form referred to herein as "Form 1", may be prepared in the form of a batch that is manufactured according to GMP which may be used as an API.

### Dosage Amounts

Compositions may be formulated to contain an effective amount of the salt of 2-Br-LSD. As used herein, the term "effective amount" refers to an amount of a 2-Br-LSD salt of the present disclosure or a composition including the 2-Br-LSD salt, effective in producing the desired response in a particular patient in need thereof. Therefore, the term "effective amount" includes the amount of a 2-Br-LSD salt of the present disclosure, that when administered, induces a positive modification in the disease or disorder to be treated, or is sufficient to prevent development or progression of the disease or disorder, or alleviate to some extent, one or more of the symptoms of the disease or disorder being treated in a subject, for example, cluster headaches and migraines.

The total daily dosage of the crystalline salt and usage thereof may be decided in accordance with standard medical practice, *e.g.*, by the attending physician using sound medical judgment. The specific therapeutically effective dose for any particular subject may depend upon a variety of factors including the disease or disorder being treated and the severity thereof (e.g., its present status); the age, body weight, general health, sex and diet of the subject; the time of administration, route of administration, and rate of excretion of the specific compound employed; the duration of the treatment; drugs used in combination or coincidental with the crystalline salt; and like factors well known in the medical arts *(see,* for example, Goodman and Gilman's The Pharmacological Basis of Therapeutics, 10th Edition, A. Gilman, J. Hardman and L. Limbird, eds., McGraw-Hill Press, 155-173, 2001).

The 2-Br-LSD salts of the present disclosure and pharmaceutical compositions thereof may be effective over a wide dosage range. *See,* for example, U.S. Patent 8,415,371.

Effective daily dosage ranges for 2-Br-LSD salts of the present disclosure are generally between about 10 µg/kg and about 100 µg/kg body weight. In some embodiments, the desired dosage may range from about 20 µg/kg to about 50 µ/kg body weight. For example, a dose of 2.4 mg may be administered to an 80 kg adult to deliver about 30 µg/kg of a 2-Br-LSD salt of the present disclosure. In some embodiments, a dose ranging from about 1.5 to 5 mg may be administered. In some embodiments, a dose ranging from about 1.5 to 3 mg may be administered.

### Pharmaceutical Kits

**The** 2-Br-LSD salts of the present disclosure and/or pharmaceutical compositions containing them may be assembled into kits or pharmaceutical systems. Kits or pharmaceutical systems according to this aspect of the disclosure include a carrier or package such as a box, carton, tube or the like, having in close confinement therein one or more containers, such as vials, tubes, ampoules, or bottles, which contain a 2-Br-LSD salt of the present disclosure or a pharmaceutical composition thereof. The kits or pharmaceutical systems of the disclosure may also include printed instructions for using the 2-Br-LSD salt and pharmaceutical compositions.

These and other aspects of the present disclosure will be further appreciated upon consideration of the following Examples, which are intended to illustrate certain particular embodiments of the disclosure but are not intended to limit its scope, as defined by the claims.

### EXAMPLES

### Example 1: General Methods.

### Solution Proton Nuclear Magnetic Resonance (¹H NMR)

¹H NMR spectra were collected using a JEOL ECX 400MHz spectrometer equipped with an auto-sampler. The samples were dissolved in a suitable deuterated solvent for analysis. The data were acquired using Delta NMR Processing and Control Software version 4.3. A Bruker Avance 300 MHz spectrometer was also used to analyze samples.

### Solution carbon-13 Nuclear Magnetic Resonance (¹³C NMR)

¹³C NMR spectra were collected using a Bruker Avance 75 MHz spectrometer. The samples were dissolved in a suitable deuterated solvent for analysis.

### X-Ray Powder Diffraction (XRPD)

X-Ray Powder Diffraction patterns were collected as described above.

### Thermo-Gravimetric Analysis (TGA)

TGA data were collected on a TA Instruments Discovery TGA equipped with a 25-position auto-sampler. The instrument was calibrated using a certified weight and certified Alumel and Nickel for temperature. A predefined amount of the sample, ca. 5 mg, was loaded into a pre-tared aluminum ACCUPIK^{™} sample pan and platinum crucible and was heated at 20 °C/min from ambient (room) temperature to 400 °C unless otherwise stated. A nitrogen purge at 25 mL/min was maintained over the sample. The instrument control, data acquisition and analysis were performed with TA Instruments TRIOS software v4.1.1.33073.

### Differential Scanning Calorimetry

DSC data was collected on a TA Instruments Discovery DSC^{®} equipped with a 50-position sample holder. The instrument was verified for energy and temperature calibration using certified indium. A predefined amount of the sample, 0.5-2.0 mg, was placed in a Tzero^{®}Pan with a Tzero^{®} Hermetic Lid and heated at 20 °C/min from 30 to 350 °C, or varied as experimentation dictated. A purge of dry nitrogen at 50 mL/min was maintained over the sample. The instrument control, data acquisition and analysis were performed with TA Instruments TRIOS software v4.1.1.33073. All results feature endotherm up convention.

### Dynamic Vapor Sorption

Sorption isotherms were obtained using a Hiden Isochema moisture sorption analyser (model IGAsorp), controlled by IGAsorp Systems Software V6.50.48. The sample was maintained at a constant temperature (25 °C) by the instrument controls. The humidity was controlled by mixing streams of dry and wet nitrogen, with a total flow of 250 mL/min. The instrument was verified for relative humidity content by measuring three calibrated Rotronic salt solutions (10 - 50 - 88%). The weight change of the sample was monitored as a function of humidity by a microbalance (accuracy +/- 0.005 mg). A defined amount of sample was placed in a tared mesh stainless steel basket under ambient conditions. A full experimental cycle typically consisted of three scans (sorption, desorption and sorption) at a constant temperature (25 °C) and 10% RH intervals over a 0 - 90% range (60 minutes for each humidity level). This type of experiment demonstrates the ability of samples studied to absorb moisture (or not) over a set of well-determined humidity ranges.

### HPLC Method 1

System: Agilentl 100 series liquid chromatograph or equivalent
Column: Xselect^{™} CSH C18 4.6 x 150 mm; 3.5 µm
(Ex. Waters^{™}; # 186005270)
Mobile Phase: A - Purified Water:MeCN:TFA(95:5:0.1)
B - MeCN:Purified water:TFA (95:5:0.1)
Flow Rate: 1.0 mL/min
Injection Volume: 5 µL
Detection: UV @ 230 nm
Column Temperature: 35 °C
Post run time: 5 minutes
Gradient:

| Time (min) | %A | %B |
|---|---|---|
| 0 | 100 | 0 |
| 3 | 100 | 0 |
| 15 | 20 | 80 |
| 20 | 20 | 80 |
| 21 | 100 | 0 |

Sample preparation: Mixture of 0.4 mg/mL in Methanol:Water (1:1) was sonicated to fully dissolve sample.

### HPLC Method 2

System: Agilent1100 series liquid chromatograph or equivalent
Mobile Phase A: acetonitrile : water (5 : 95) + trifluoroacetic acid 0.05%
Mobile Phase B: acetonitrile : water (95 : 5) + trifluoroacetic acid 0.05%
Injection Volume: 5 µL
Flow: 1 mL/min
Column: Kinetex^{®} 2.6 µm XB-C18 100 Å, 100 x 4.6 mm
Column Temperature: 35 °C
Autosampler Temperature: 4 °C
Detection: UV 240 nm
Gradient:

| Time (min) | % A | % B |
|---|---|---|
| Start | 100.0 | 0.0 |
| 3.00 | 100.0 | 0.0 |
| 15.00 | 20.0 | 80.0 |
| 20.00 | 20.0 | 80.0 |
| 23.00 | 100.0 | 0.0 |

Sample preparation: Weigh about 25 mg of powder and dilute to 100 mL with Mobile Phase A : Mobile Phase B 60 : 40

### Example 2: Preparation of crystalline 2-Br-LSD hydrobromide (Form 1).

### Method 1

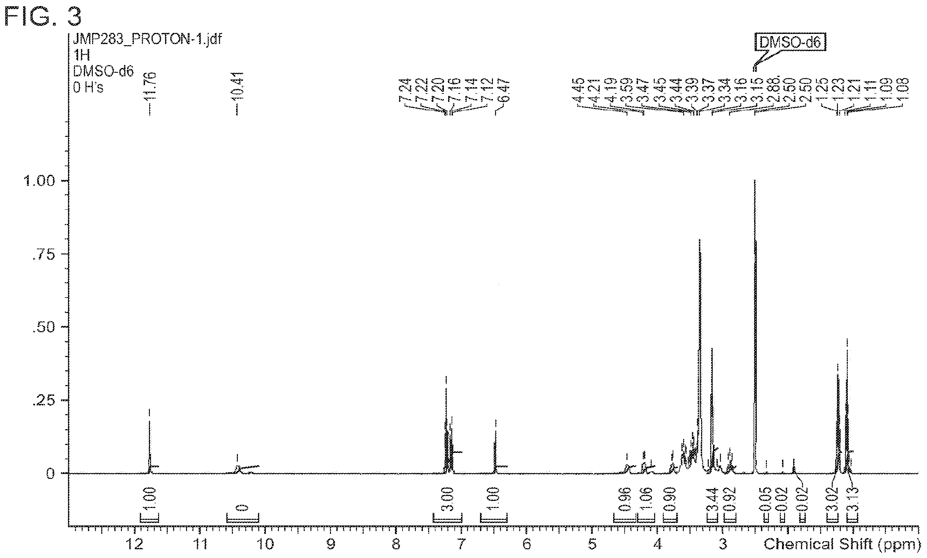

Hydrobromic acid (1M solution in ethanol) was prepared by diluting 172.15 g of HBr_{aq} 47% w/w with ethanol to a final volume of 1000 mL, mixing well, and allowing the solution to equilibrate to ambient temperature. 2-Br-LSD free base (5.0 g, 0.012 mol, 1 equiv) was charged to a flask and ethanol (50 mL, 10 volumes) was added. The mixture was heated to 50 °C to give a yellow solution. Hydrobromic acid (1M solution in ethanol, 12.43 mL, 1 equiv) was added dropwise. Precipitation was noted following roughly 50% of the acid charge. The suspended solid was isolated via rapid evaporation *in vacuo* producing an off-white solid. The solid was dried *in vacuo* at 50 °C for 18 hours to afford 2-Br-LSD hydrobromide (Form 1) as an off-white solid (5.48 g, 91% yield). 22-Br-LSD hydrobromide (Form 1) was successfully prepared, with the XRPD pattern (FIG. 1) and thermal profile (FIG. 2) conforming to previous examples of the salt. The XRPD peak list is presented in Table 2A. The DSC curve (FIG. 2) shows no evidence of a liquid phase prior to exothermic decomposition at 259 °C. The corresponding TGA curve (FIG. 2) shows little or no weight reduction until decomposition. The ¹H NMR spectrum is consistent with the proposed structure with the presence of 0.4% residual ethanol (FIG. 3).

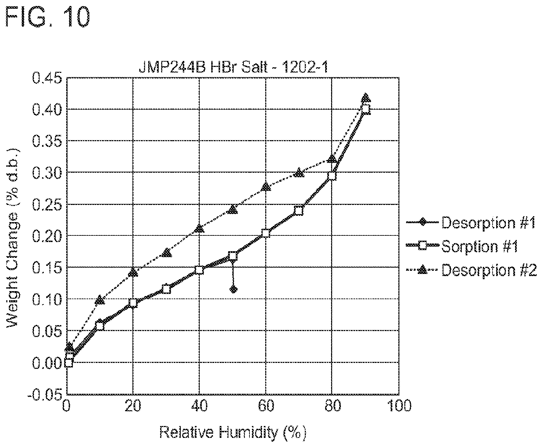

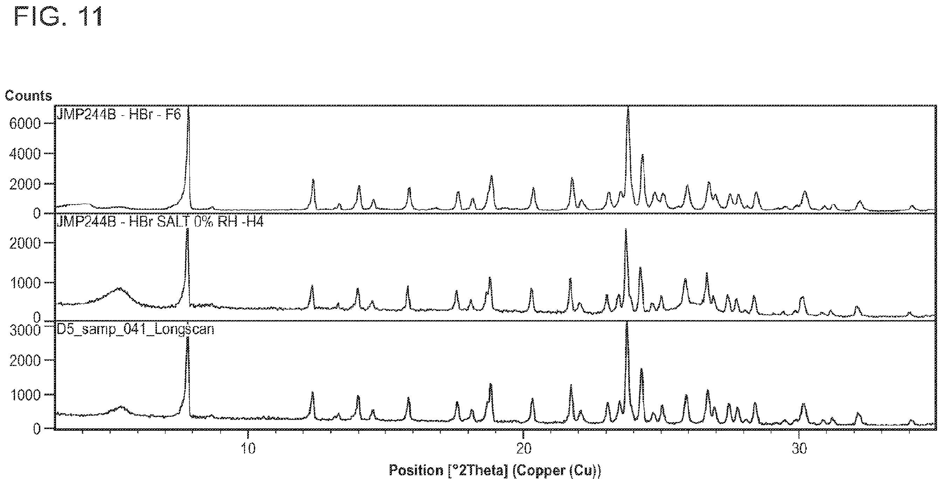

2-Br-LSD hydrobromide (Form 1) is non-hygroscopic. After drying *in vacuo* at 50 °C for 18 hours, it exhibited less than 1% weight loss between 25 °C and 200 °C upon thermogravimetric analysis at a heating rate of 20 °C/min (FIG. 2). Also, it exhibited less than about 0.5% weight change upon dynamic vapor sorption analysis (FIG. 10) at 25 °C over the relative humidity (RH) range 0-90% when measured at 10% RH intervals and 60 minutes for each humidity level. During the initial sorption cycle the solid gained 0.21% of water up to 60%RH with a further 0.2wt% increase noted between 60%RH and 90%RH resulting in a total of 0.4%. This water uptake was likely to relate to surface wetting of the solid. The water uptake was reversible as no water was retained at 0% RH. Minor hysteresis was noted. XRPD analysis of the 2-Br-LSD hydrobromide (Form 1) showed that no form change occurred following equilibration at extremes of humidity. Thus, FIG. 11 is a comparison of the XRPD patterns of 2-Br-LSD hydrobromide (Form 1) (top) ex DVS following equilibration at 0% relative humidity (middle) and 90% relative humidity (bottom).

### Method 2

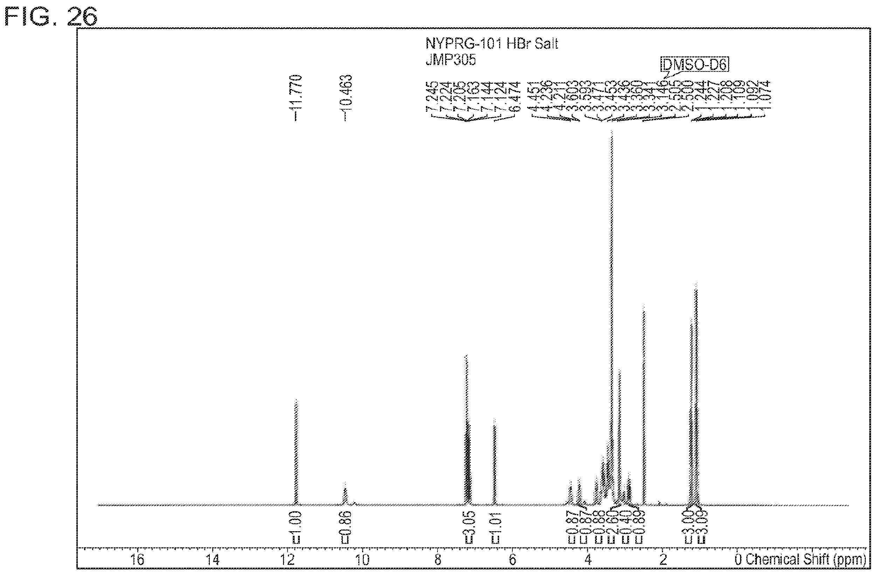

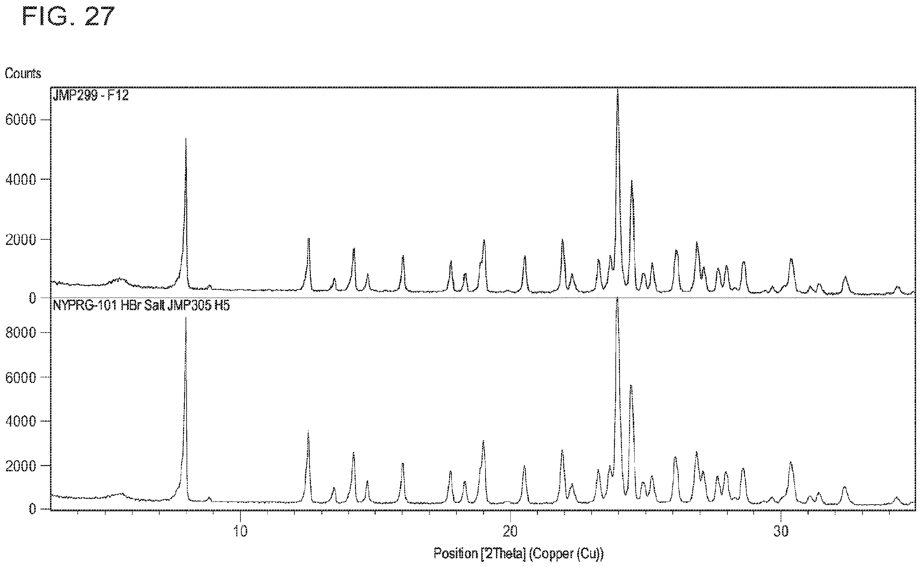

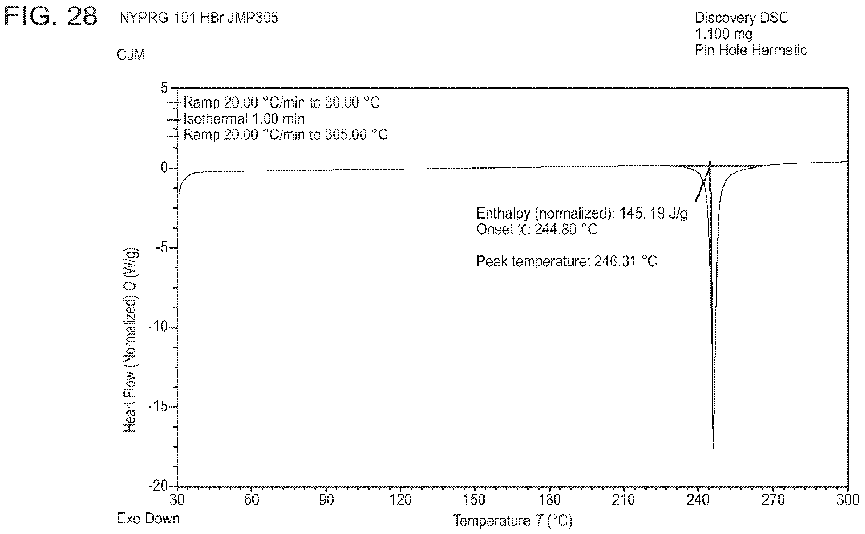

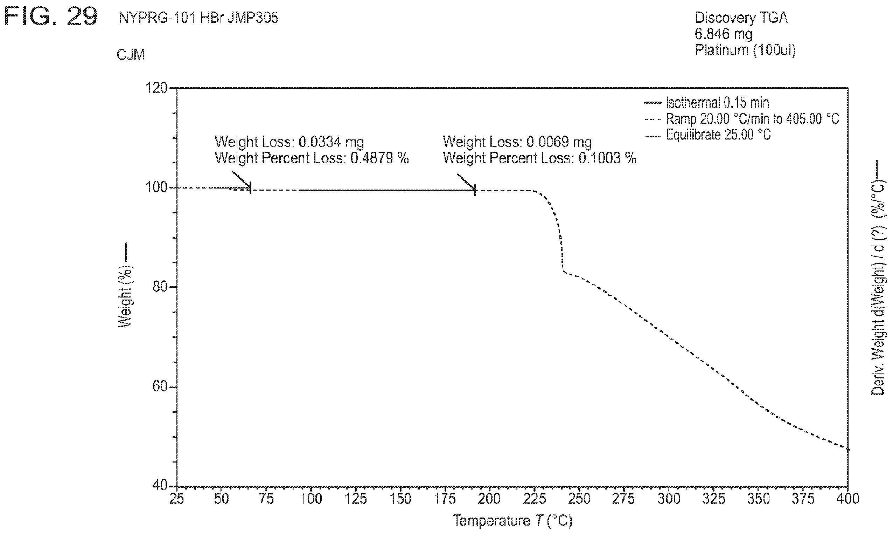

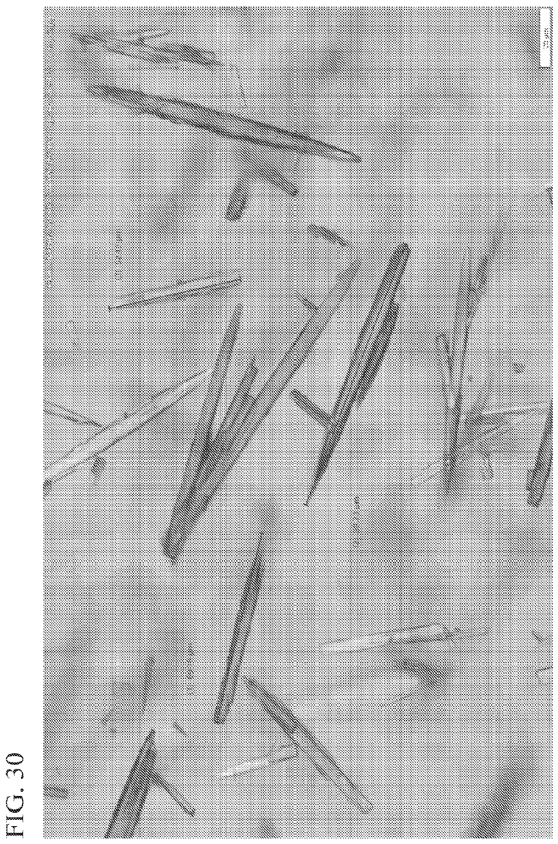

A 2M stock solution of hydrobromic acid in ethanol-water 95:5 was prepared by diluting 48% aqueous HBr with ethanol and deionized water to volume. 2-Br-LSD (48 g) was weighed into a 1000 mL jacketed reaction vessel fitted with overhead stirring. Ethanol (506.4 mL) and deionized (DI) water (17.5 mL) were charged to the vessel, and the mixture was stirred at ambient temperature with an agitation rate of 200 rpm for about 30 minutes to give a dark brown solution. Hydrobromic acid (2M in ethanol-water 95:5 v/v, 12.2 mL) was added in one portion, followed immediately by seeding with 2-Br-LSD hydrobromide (Form 1) (0.06 g, 0.1% w/w). Stirring was continued at ambient temperature for 2 hours after the addition, whereupon the mixture showed visible signs of crystallization development. Hydrobromic acid (2M in ethanol-water 95:5, 48.7 mL) was added over 4 hours using a syringe pump. Stirring was continued for 1 hour at ambient temperature. The mixture was cooled to 5 °C over 2 hours and stirred at this temperature for 18 hours. The solid was isolated using vacuum filtration. Ethanol-water 95:5 (57 mL) was used to rinse the vessel and passed through the damp cake. The cake was pulled dry under air/vacuum for 30 minutes. The resulting solid was dried *in vacuo* at 45 °C for 60 hours to afford 51 g (89% yield, Lot JMP305). HPLC analysis by Method 1 showed 99.28% purity by area. The ¹H NMR spectrum (FIG. 26) is in agreement with 2-Br-LSD hydrobromide containing 0.3% residual ethanol. The powder x-ray diffractogram in FIG. 27 (lower trace), collected on a PANalytical X'pert PRO diffractometer using Cu Kα radiation as described above, is in agreement with the reference pattern for 2-Br-LSD hydrobromide (Form 1) shown in FIG. 1, as well as with the reference pattern shown in the upper trace in FIG. 27. Differential scanning calorimetry (DSC, FIG. 28) and thermogravimetric analysis (TGA, FIG. 29) are typical of 2-Br-LSD-hydrobromide (Form 1), exhibiting no evidence of a liquid phase prior to exothermic decomposition at 246 °C, and are consistent with loss of residual ethanol. Microscopy (FIG. 30, 50X objective, sample dispersed in oil) shows a rod-shaped crystal habit with some acicular nature, 50-100 microns in length. Taken together, the analytical data support the assignment of Lot JMP305 as 2-Br-LSD hydrobromide (Form 1).

### Method 3

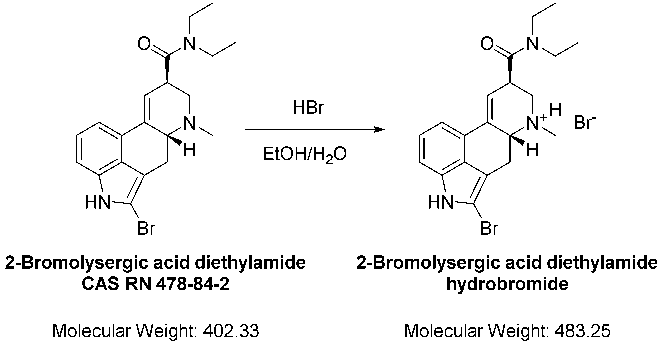

| LIST OF STARTING MATERIALS AND REAGENTS: | | | | |
|---|---|---|---|---|
| **Material** | **Amount** | **MW** | **mmol** | **Ratio** |
| 2-Br-LSD | 150.0 g | 402.33 | 372.8 | 1.0 equivalent |
| Denatured ethanol | 1275 mL | | | 8.5 volumes |
| Demineralized water | 67.5 mL | | | 0.45 volumes |
| HBr_{aq} 47% w/w | 12.8 g | 80.91 | 74.3 | 0.2 equiv |
| Denatured ethanol | 27.3 mL | | | 0.18 volumes |
| Demineralized water | 1.4 mL | | | 0.009 volumes |
| 2-bromolysergic acid diethylamide hydrobromide | 0.15 g | 483.24 | 3.1 x 10⁻⁴ | 0.1% w/w |
| HBr_{aq.} 47% w/w | 51.5 g | 80.91 | 298.5 | 0.8 equiv |
| Denatured ethanol | 109.1 mL | | | 0.73 volumes |
| Demineralized water | 5.7 mL | | | 0.04 volumes |
| Denatured ethanol | 142.5 mL | | | 0.95 volumes |
| Demineralized water | 7.5 mL | | | 0.05 volumes |

In a suitable reactor, 2-Br-LSD (150.0 g) was charged, followed by denatured ethanol (1275 mL) and water (67.5 mL). The total solvent volume (1342.5 mL) corresponded to 8.95 volumes and an ethanol/water ratio of 95:5. The mixture was heated to 45 °C, resulting in complete dissolution. Aqueous (aq.) HBr (2 M) was prepared in a 200 mL volumetric flask by bringing to volume 68.8 g of aq. HBr 47% w/w with a 95:5 mixture of denatured ethanol and water to obtain a concentration of 2 M. The solution was homogenized, and then 37.3 mL of this solution (0.2 equiv, consisting of 12.8 g of aq. HBr 47% w/w, 27.3 mL of denatured ethanol and 1.4 mL of water) was added to the reaction vessel via a dropping funnel over 80 minutes while maintaining an internal temperature of 45 °C. At the end of the addition, the mixture was seeded with 0.15 g of 2-bromolysergic acid diethylamide hydrobromide and stirred for two hours at 45 °C. Aqueous HBr (2 M) 149.3 mL (0.8 equiv, consisting of 51.4 g of HBr_{aq} 47% w/w, 109.1 mL of denatured ethanol and 5.7 mL of water) was added to the reaction vessel via a dropping funnel over 180 minutes while maintaining an internal temperature of 45 °C. The mixture was allowed to spontaneously cool to room temperature (20 - 25 °C) and stirred overnight. The resulting suspension was then cooled to 0 - 5 °C and stirred for 60 minutes. The product was collected on a Büchner filter, washed with denatured ethanol/water (95:5, 142.5 mL of ethanol, 7.5 mL of water) and dried in a vacuum oven at 45 °C for 18 hours to give 127.5 g (70.7% molar yield, 85.0% w/w yield).

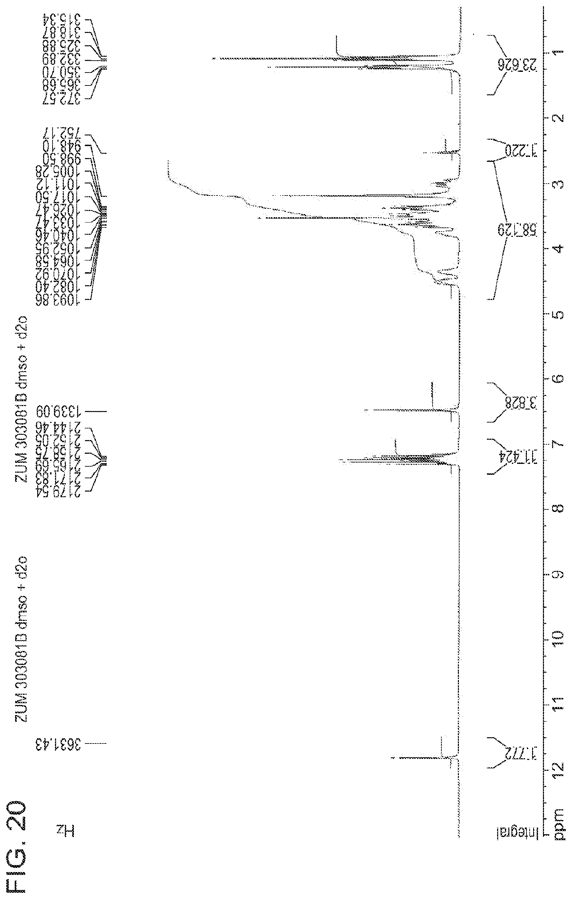

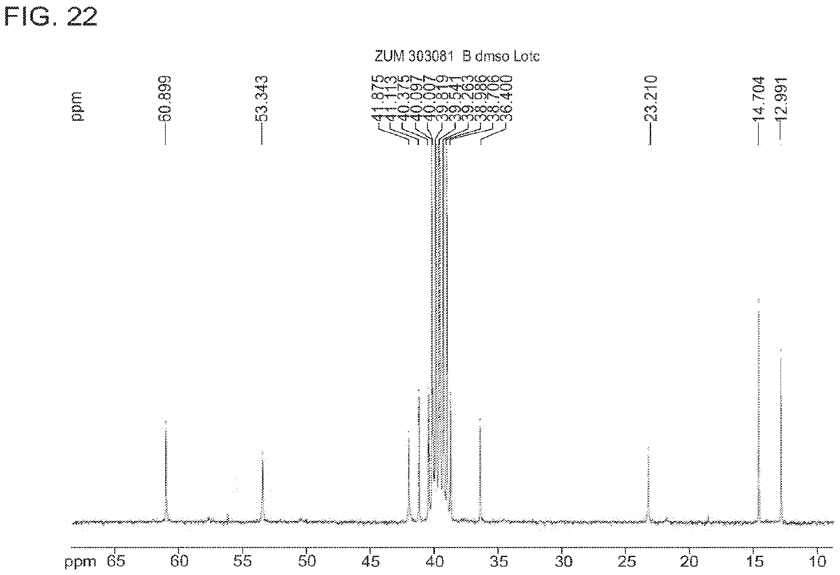

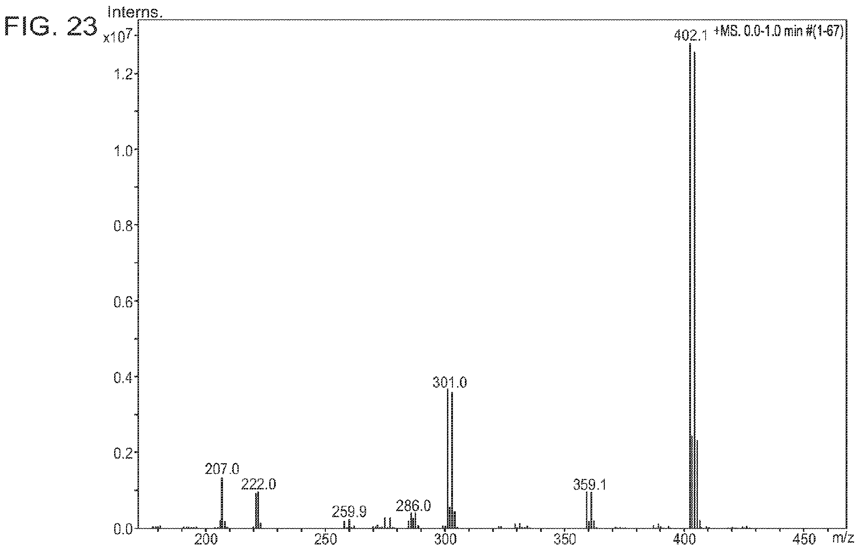

Analysis by HPLC Method 2 indicated 99.45% 2-bromolysergic acid diethylamide by area. The ¹H NMR spectra (FIG. 19 and FIG. 20), ¹³C NMR spectra (FIG. 21 and FIG. 22) and mass spectrum (FIG. 23, Agilent 6330 Ion Trap, [M+H]⁺ 402.17) are all in agreement with the proposed structure. The powder x-ray diffractogram in FIG. 24, collected using a Bruker D2 X-ray Generator (30 kV, 10 mA, scan interval 3-35°, Step 0.04°, 2 sec/step; flat plate zero-background sample holder, rotation 15 rpm), is in agreement with the reference pattern for 2-Br-LSD hydrobromide (Form 1) shown in FIG. 1. Differential scanning calorimetry (DSC): onset at 244 °C (see, FIG. 25). Moisture by Karl Fischer titration (KF): 0.11%. Hydrogen bromide by potentiometric titration with NaOH: 16.1% (calculated for C₂₀H₂₄BrN₃O.HBr: 16.7%). Residue on ignition (ROI): 0%. Total residual solvents: 0.36% w/w. Potency = (100 - KF - residual solvents - ROI) x HPLC purity = 98.98%.

### Method 4

The following process was performed in accordance with GMP appropriate for early-stage clinical use as defined in Q7. All processing steps were conducted under an atmosphere of nitrogen, unless indicated otherwise.

A clean, dry reactor was subjected to three vacuum (≤400 mbar) - nitrogen purge cycles. 2-Bromo-LSD (2.320 kg, prepared as described in Example 5 (STEPS 1 and 2) of Applicant's copending application, filed on even date herewith, and entitled "PROCESS FOR SYNTHESIZING 2-BROMOLYSERGIC ACID DIETHYLAMIDE VIA CONTROLLED HYDROLYSIS OF BROMOCRIPTINE"; Attorney Docket No. 58205-00003PCT), was charged through the hatch, followed by three vacuum (≤400 mbar) - nitrogen purge cycles. A second clean, dry reactor was subjected to three vacuum (≤400 mbar) - nitrogen purge cycles. Denatured ethanol (15.5 kg) and purified water (1.0 L) were charged to the second reactor, followed by three vacuum (≤400 mbar) - nitrogen purge cycles, and this mixture was agitated for not less than 5 minutes. The ethanol-water mixture was transferred to the reactor containing 2-Br-LSD, agitation was begun, and the resulting mixture was heated to 43 - 47 °C, resulting in complete dissolution of the solid. Inside a glove box, denatured ethanol (0.3 kg), purified water (0.02 L) and hydrobromic acid 48% (0.2 kg) were measured out and charged to a dropping funnel. The contents of the dropping funnel were added in not less than 120 minutes to the stirred 2-Br-LSD solution while maintaining an internal temperature of 43 - 47 °C. Stirring was continued at this temperature for not less than 2 hours. Inside the glove box, denatured ethanol (1.3 kg), purified water (0.1 L) and hydrobromic acid 48% (0.8 kg) were measured out and charged to the dropping funnel. The contents of the dropping funnel were added in not less than 180 minutes to the batch while maintaining an internal temperature of 43 - 47 °C. With continuous agitation, the batch was allowed to cool spontaneously to an internal temperature of 20 - 24 °C and maintained in this range for 18 hours. The batch was cooled to 0 - 5 °C, stirred for 60 minutes, and filtered. Purified water (0.1 L) and denatured ethanol (1.7 kg) were charged to the reactor, and this mixture was cooled to 0 - 5 °C and used to wash the solid on the filter. The solid was dried in a vacuum oven at 43-47 °C for not less than 18 hours until in-process controls (IPCs) for residual solvents (≤ ICH limits for solvents used in the process) and moisture by Karl Fischer titration (KF; ≤0.5%) were met, to give 2.410 kg of off-white powder.

The product was characterized and released in accordance with GMP for early-stage clinical use as defined in Q7. Selected acceptance specifications and results are presented in Table 9. Where applicable, the results were compared to the product prepared and characterized as described in Example 2, Method 3, and which in Table 9 and the paragraph following Table 10, is referred to as a "reference standard."

**Table 9. Selected Release Testing Results for 2-Br-LSD hydrobromide (Form 1) Prepared According to Method 4**

| **Test** | | **Specification** | **Result** |
|---|---|---|---|
| **Selected Release Tests** | | | |
| Appearance | | Off-white to light brown powder | Complies |
| Identification | | | |
| | Infrared spectrum | Conforms with reference spectrum | Complies *^{a}* |
| | HPLC retention time *^{b}* | Retention time of main peak is ±5% of retention time of reference standard | Complies |
| HBr content by potentiometric titration (% w/w on anhydrous basis) | | 15.7 - 17.7% | 16.8% |
| Assay by HPLC (% w/w against reference standard, on anhydrous, solvent-free basis) *^{b}* | | 97.0 - 102.0% | 99.7% |
| Chemical purity by HPLC (% a/a) *^{b}* | | NLT 98.0% | 99.4% |
| Related substances by HPLC (% a/a) *^{b}* | | | |
| | LSD *^{c}* | NMT 0.05% | ND *^{d}* |
| | RRT 0.99 *^{e}* | NMT 0.50% | 0.25% |
| | Iso-2-Br-LSD (RRT 1.16)*^{f}* | NMT 0.15% | ND *^{g}* |
| | Largest single unknown or unqualified impurity | NMT 0.15% | RRT 0.86 = 0.11% |
| | | | RRT 1.17 = 0.05% |
| | Report all impurities greater than LOQ (0.05%) | | RRT 1.21 = 0.13% |
| | | | RRT 1.38 = 0.07% |
| | Total impurities | NMT 2.0% | 0.6% |
| Residual solvents by HS-GC | | | |
| | Methanol | NMT 3000 ppm | 10 ppm |
| | Dichloromethane | NMT 600 ppm | 10 ppm |
| | Acetonitrile | NMT 410 ppm | 4 ppm |
| | Diethylamine | NMT 1000 ppm | 90 ppm |
| | Ethyl acetate | NMT 5000 ppm | 13 ppm |
| | Ethanol | NMT 5000 ppm | 2471 ppm |
| | Acetone | NMT 5000 ppm | 132 ppm |
| | 2-Propanol | NMT 5000 ppm | 115 ppm |
| Water content by KF (% w/w) | | NMT 0.5% | 0.1% |
| Residue on ignition (% w/w) | | NMT 0.5% | 0.0% |
| XRPD | | Conforms to Form 1 reference diffractogram | Complies *^{h}* |

| **Additional Tests for Information Only** | | | |
|---|---|---|---|
| Identification | | | |
| | ¹H NMR | Report result - compare to reference spectrum | Conforms |
| | Mass spectrometry | Report result | [M+H]⁺ 402.6 |
| *^{a}* FIG. 46 | | | |
| *^{b}* HPLC Method 2, except that diluent is Mobile Phase A : Mobile Phase B 40 : 60 | | | |
| *^{c}* (6aR,9R)-N,N-diethyl-7-methyl-4,6,6a,7,8,9-hexahydroindolo[4,3-fg]quinoline-9-carboxamide | | | |
| *^{d}* No peak assignable to LSD was detected by HPLC with UV absorbance detection at 240 nm with LOD 0.02% and ion trap mass detection at m/z 324 ([M+H]⁺) | | | |
| *^{e}* Tentatively assigned as (6aR)-5-bromo-N,N-diethyl-7-methyl-4,6,6a,7,8,10a-hexahydroindolo[4,3-fg]quinoline-9-carboxamide | | | |
| *^{f}* (6aR,9S)-5-bromo-N,N-diethyl-7-methyl-4,6,6a,7,8,9-hexahydroindolo[4,3-fg]quinoline-9-carboxamide | | | |
| *^{g}* LOD 0.02% | | | |
| *^{h}* FIG. 47 | | | |
| % w/w = % weight-to-weight; % a/a = % of total peak area; NMT = not more than; NLT = not less than; RRT = relative retention time; ND = not detected; LOD = limit of detection; HPLC = high performance liquid chromatography; HS-GC = headspace gas chromatography; ppm = parts per million; KF = Karl Fischer moisture titration; XRPD = x-ray powder diffraction; NMR = nuclear magnetic resonance spectroscopy | | | |

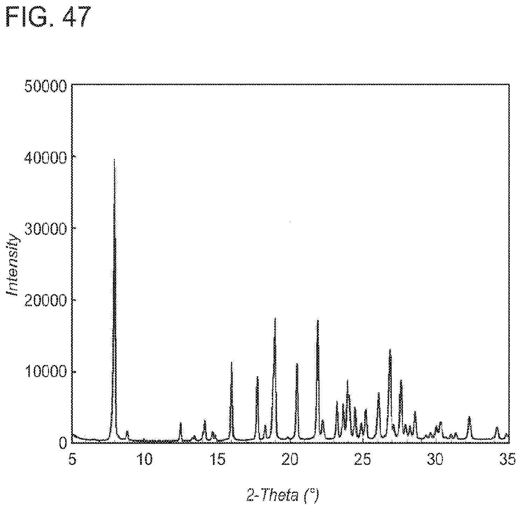

The XRPD pattern for 2-bromolysergic acid diethylamide hydrobromide (Form 1) prepared according to Method 4 was collected using a Bruker D2 X-ray Generator (30 kV, 10 mA, scan interval 3-35°, Step 0.03°, 2 sec/step; flat plate zero-background sample holder, rotation 15 rpm). The results are presented graphically in FIG. 47 and in tabular form in Table 10.

**Table 10. XRPD Diffraction Peaks for 2-Bromolysergic acid diethylamide hydrobromide (Form 1) Prepared According to Method 4**

| Pos. [°2Th.] | Rel. Int. [%] |
|---|---|
| 8.0 ± 0.2 | 100.0 |
| 8.9 ± 0.2 | 4.3 |
| 12.5 ± 0.2 | 7.2 |
| 14.2 ± 0.2 | 8.2 |
| 14.7 ± 0.2 | 4.2 |
| 16.0 ± 0.2 | 28.7 |
| 17.8 ± 0.2 | 23.1 |
| 18.3 ± 0.2 | 6.5 |
| 19.0 ± 0.2 | 43.0 |
| 20.5 ± 0.2 | 29.2 |
| 21.9 ± 0.2 | 43.1 |
| 22.2 ± 0.2 | 8.4 |
| 23.2 ± 0.2 | 14.9 |
| 23.6 ± 0.2 | 13.6 |
| 23.9 ± 0.2 | 22.2 |
| 24.4 ± 0.2 | 13.1 |
| 24.9 ± 0.2 | 7.1 |
| 25.2 ± 0.2 | 12.0 |
| 26.1 ± 0.2 | 18.2 |
| 26.8 ± 0.2 | 32.6 |
| 27.1 ± 0.2 | 6.6 |
| 27.6 ± 0.2 | 22.7 |
| 27.9 ± 0.2 | 6.4 |
| 28.2 ± 0.2 | 6.2 |
| 28.6 ± 0.2 | 11.7 |
| 30.0 ± 0.2 | 6.1 |
| 30.3 ± 0.2 | 7.5 |
| 31.3 ± 0.2 | 3.8 |
| 32.3 ± 0.2 | 9.4 |
| 34.2 ± 0.2 | 5.4 |
| 36.0 ± 0.2 | 7.0 |
| 38.2 ± 0.2 | 9.3 |

This XRPD pattern is consistent with that of the reference standard prepared according to Example 2, Method 3, and with those presented in FIG. 1, FIG. 24, FIG. 27, Table 2A and Table 2D for 2-Br-LSD hydrobromide (Form 1).

### Example 3: Preparation of crystalline 2-Br-LSD hydrobromide (Pattern B Solvate).

2-Br-LSD hydrobromide (Form 1) (50 mg) was weighed into a crystallizations tube. Tetrahydrofuran-water (98:2 v/v; 0.500 mL, 10 volumes) was added and the mixture equilibrated at 25 °C for 24 hours. The solids were isolated by filtration and dried at 45 °C *in vacuo* to afford 2-Br-LSD hydrobromide (Pattern B Solvate), for which the XRP diffractogram and peak list are presented in FIG. 4 and Table 2B, respectively.

### Example 4: Preparation of crystalline 2-Br-LSD hydrobromide (Form 2).

2-Br-LSD hydrobromide (Form 1) (50 mg) was weighed into a crystallization tube. Ethyl acetate (0.500 mL, 10 volumes) was added, and the mixture equilibrated at 25 °C for 24 hours. The solid was isolated by filtration and dried at 45 °C *in vacuo* to afford 2-Br-LSD hydrobromide (Form 2), for which the XRP diffractogram and peak list are presented in FIG. 5 and Table 2C, respectively.

### Example 5: Preparation of crystalline 2-Br-LSD mesylate (Amorphous)

2-Br-LSD freebase (5.0 g) was charged to a flask and IPA (50 mL, 10 volumes) was added. The mixture was heated to 50 °C and allowed to equilibrate for ~ 15 minutes, to give a yellow solution. Methane sulfonic acid (1M solution in ethanol, 12.43 mL, 1 equivalent) was added in one single charge. The solvent was then flash evaporated to give a light beige solid which was dried *in vacuo* at 45 °C for 24 hours to afford a beige solid (6.10 g, 99% yield). The XRP diffractogram, ¹H NMR spectrum and DSC and TGA curves (not shown) support the assignment of this product as amorphous 2-Br-LSD mesylate.

### Example 6: Preparation of crystalline 2-Br-LSD mesylate (Form A)

2-Br-LSD mesylate (50 mg, amorphous) was weighed into a crystallization tube, followed by 1,4-dioxane (1.00 mL, 20 volumes). The mixture was heated to 40 °C and equilibrated for ~ 15 minutes. Tert-Butyl methyl ether (0.05 mL, 1 volume) was charged to the solution. The mixture was equilibrated for 5 minutes. A suspension was observed. The mixture was left to cool to ambient temperature and equilibrated for 12 hours. The solid was isolated by vacuum filtration and dried at 45 °C for 24 hours. The XRP diffractogram (FIG. 7) and XRP peak list (Table 1A) are presented as FIG. 7 and Table 1A, respectively.

### Example 7: Preparation of crystalline 2-Br-LSD mesylate (Form B)

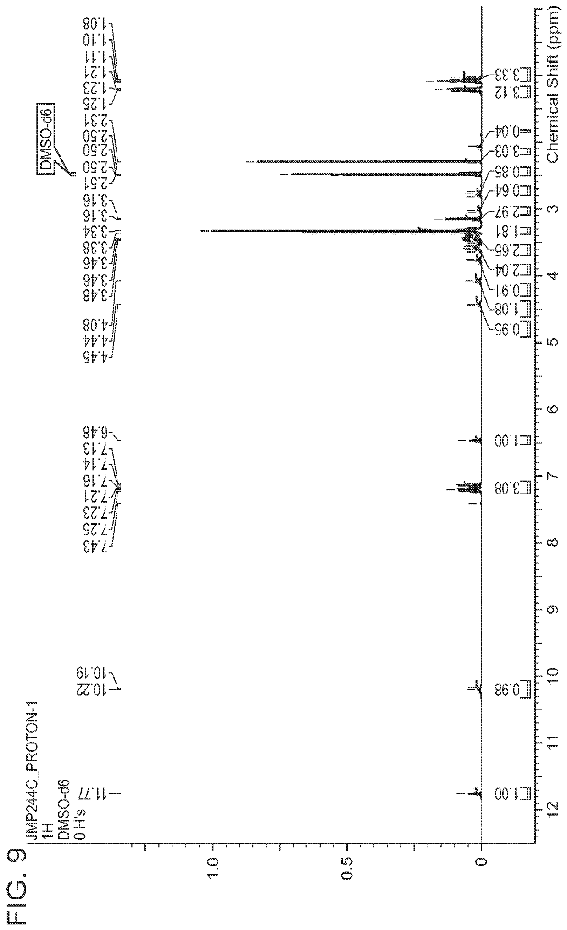

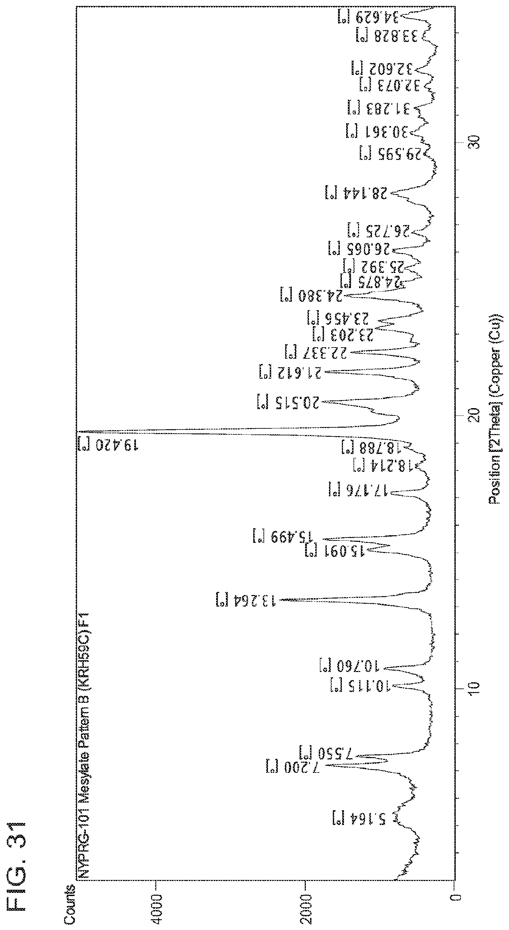

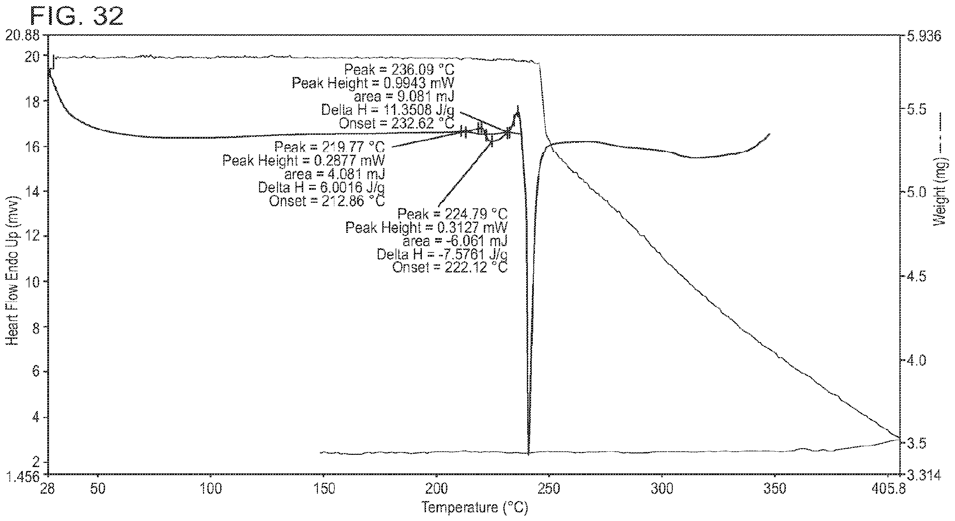

2-Br-LSD mesylate (50 mg, amorphous) was weighed into a crystallization tube and charged with tetrahydrofuran-water (98:2, 0.25 mL, 5 volumes). The mixture was heated to 40 °C and equilibrated for ~ 15 minutes. Ethyl acetate (0.30 mL) was charged to the solution in 0.05 mL (1 volume) aliquots, with equilibrations of 5 minutes after each addition. The mixture was left to cool to ambient temperature and equilibrated for 12 hours. The solid was isolated by vacuum filtration and dried at 45 °C for 24 hours. The XRP diffractogram, XRP peak list, combined DSC/TGA curves are presented as FIG. 31, Table 1B, and FIG. 32, respectively. The ¹H NMR spectrum (FIG. 9) confirmed a successful salt formation with mono stoichiometry as a methanesulfonic acid signal was present at 2.3 ppm (3H).

### Example 8: Preparation of crystalline 2-Br-LSD mesylate (Form C)

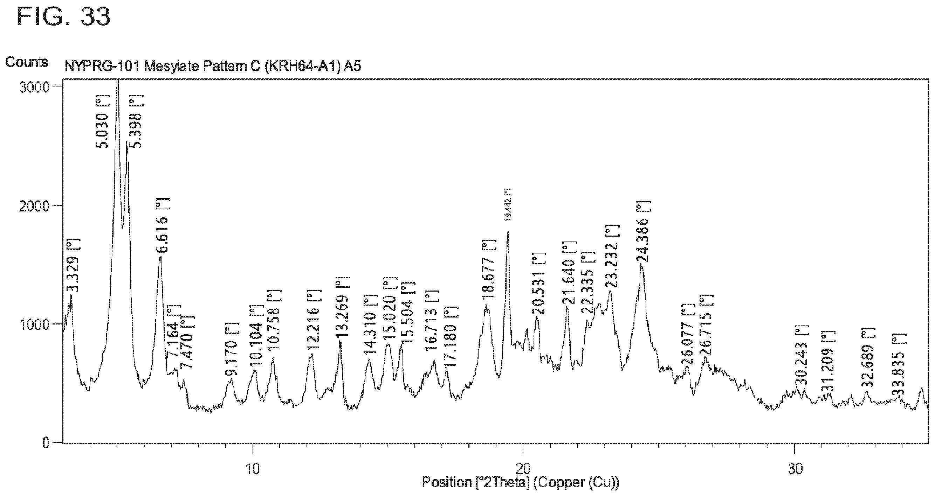

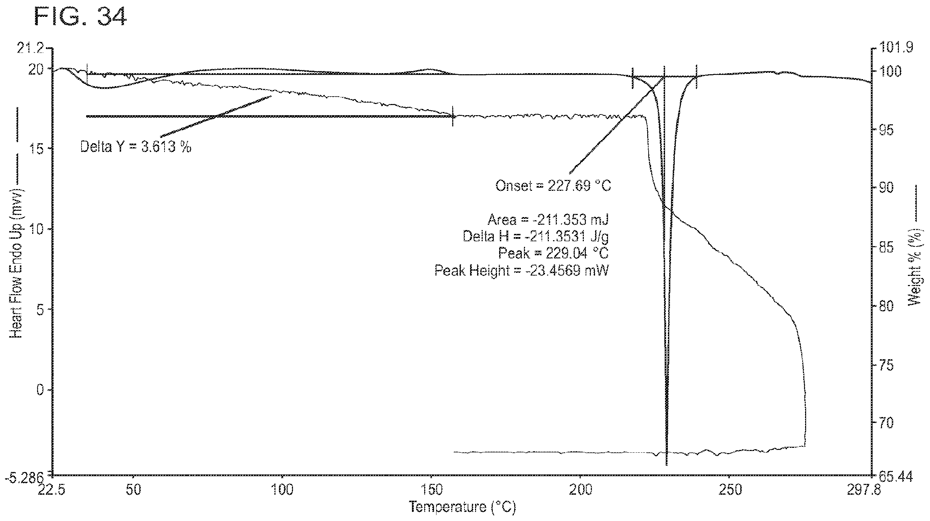

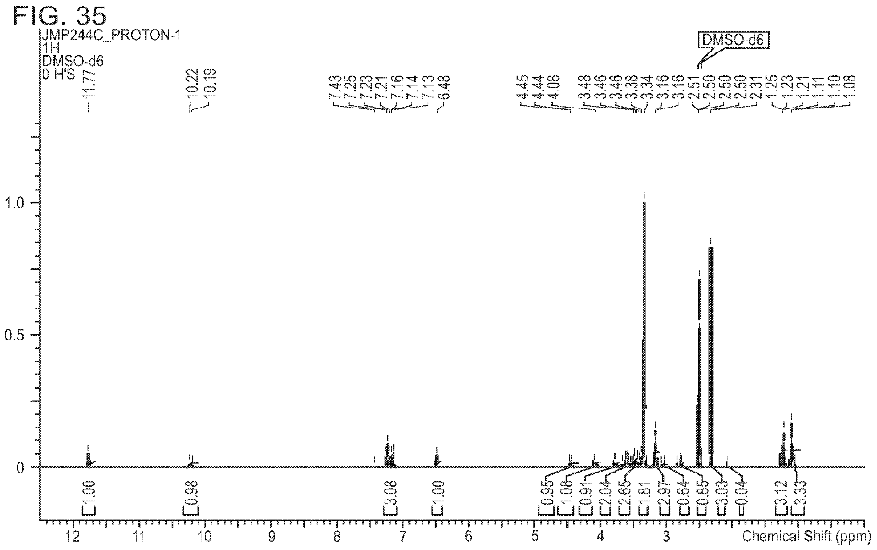

2-Br-LSD mesylate (200 mg) was weighed into a 14-mL type 1 glass vial. A minimum volume of ethanol was charged, and the mixture was heated to 50 °C. Once a solution was obtained, it was filtered to ensure the absence of any visible particulates. One fourth of this solution, containing 50 mg of 2-Br-LSD mesylate, was charged to a crystallization tube containing 1 mL of pre-cooled (2 °C) toluene. The mixture was allowed to equilibrate for ~ 30 minutes at 2 °C, resulting in a suspension. The solid was collected by vacuum filtration and dried *in vacuo* at 45 °C for 24 hours. The XRP diffractogram, XRP peak list, and combined DSC/TGA curves are presented as FIG. 33, Table 1C, and FIG. 34, respectively. The ¹H NMR spectrum (FIG. 35) confirmed a successful salt formation with mono stoichiometry as a methanesulfonic acid signal was present at 2.3 ppm (3H).

### Example 9: Preparation of crystalline 2-Br-LSD hemi-fumarate (Form A)

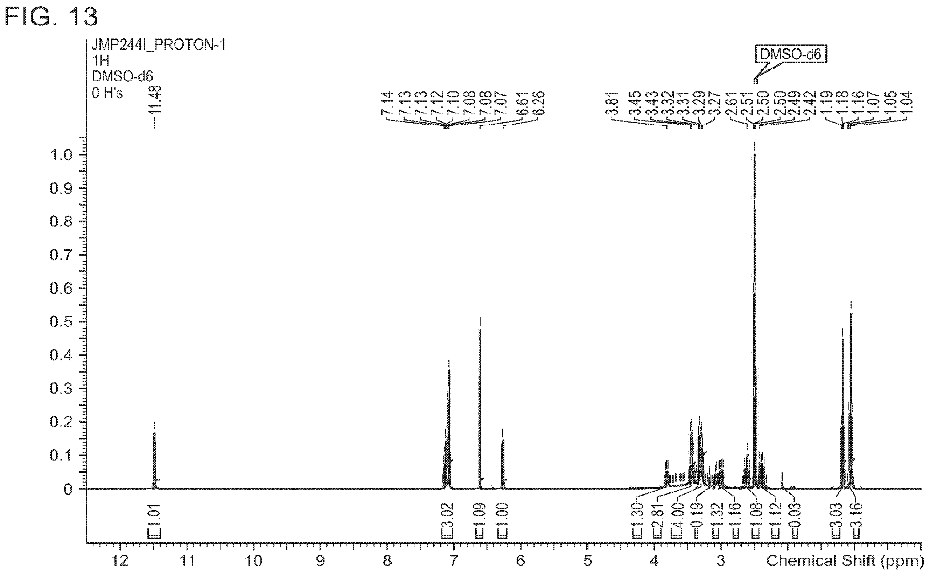

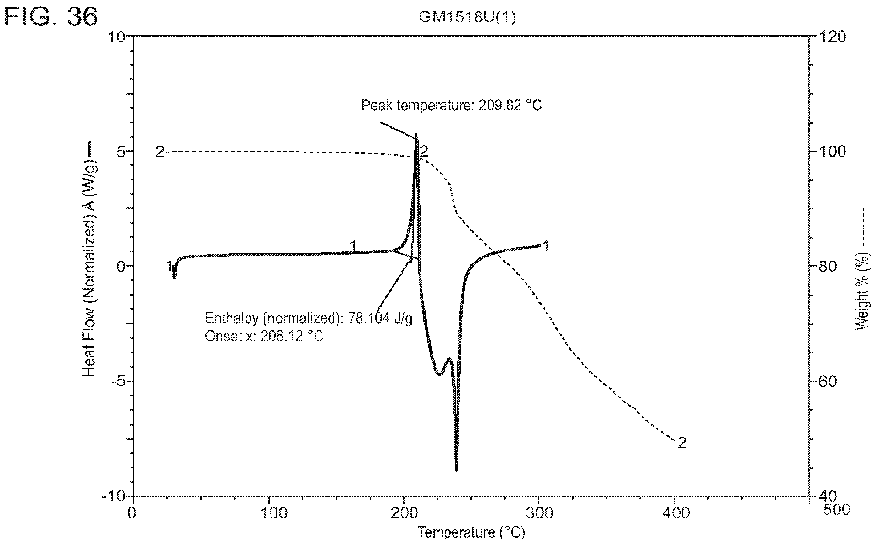

2-Br-LSD hemi-fumarate (Form B) (50 mg) was charged to a crystallization tube and suspended in 0.500 mL (10 volumes) of acetone. The mixture was heated to 50 °C and stirred for ca. 15 mins. MeOH (0.550 mL, 11 volumes) was charged to the solution at 50 °C in 0.05-mL (1 vol) aliquots with equilibrations for 5 minutes after each addition until a solution formed. The solution was clarified by filtration into a clean pre-heated tube. The mixture was equilibrated at 50 °C for about 30 mins and then cooled to 25 °C and equilibrated for about 20 hours. The resulting suspension was filtered, and the collected solid dried *in vacuo* at 45 °C. The ¹H NMR spectrum (FIG. 13) confirms a successful salt formation with hemi stoichiometry as a fumaric acid signal is present at 6.5 ppm (1H). The DSC curve (FIG. 36) of the salt shows a clear melt endotherm at 210 °C followed by an exothermic event presumably associated with decomposition. The corresponding TGA curve (FIG. 36) confirms an anhydrous solid. The XRP diffactogram and peak list are presented in FIG. 6 and Table 5A, respectively.

### Example 10: Preparation of crystalline 2-Br-LSD hemi-fumarate (Form B)

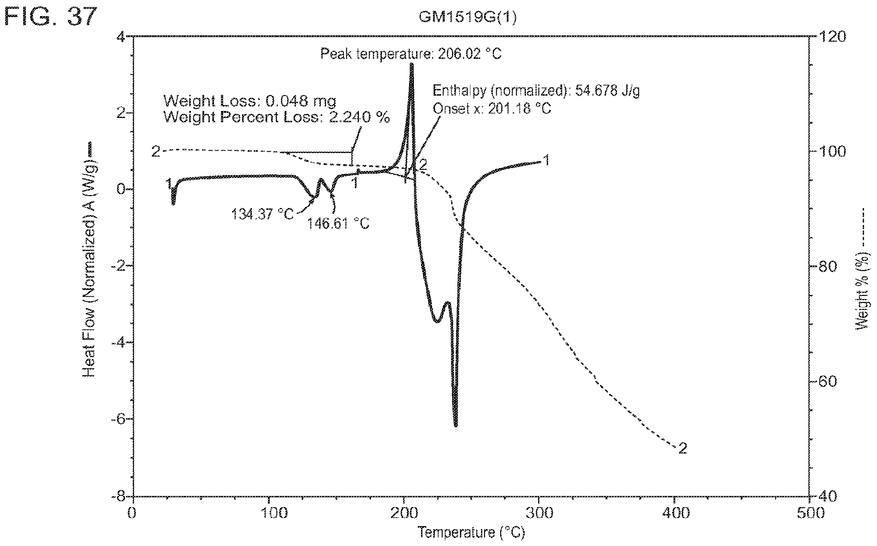

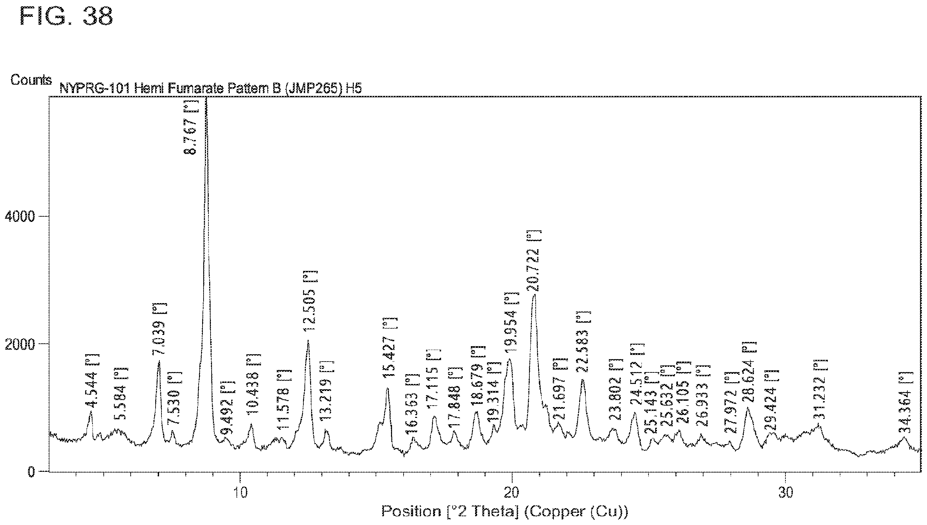

2-Br-LSD free base (5.0 g, 0.012 mol, 1 equiv) was charged to a flask and THF (50 mL, 10 volumes) was added. The mixture was heated to 50 °C to give a yellow solution. Fumaric acid (0.25 M solution in ethanol, 24.8 mL, 0.0062 mol, 0.5 equiv) was added, giving a yellow solution. The solvent was evaporated rapidly *in vacuo,* producing a pale-yellow solid, which was dried *in vacuo* at 50 °C for 18 hours to afford an off-white solid (5.4 g, 94% yield). The XRP diffractogram, XRP peak list, and combined DSC/TGA curves are presented as FIG. 38, Table 5B, and FIG. 37, respectively. The ¹H NMR spectrum (not shown) confirms a successful salt formation with hemi stoichiometry as a fumaric acid signal is present at 6.5 ppm (1H).

### Example 11: Preparation of crystalline 2-Br-LSD besylate

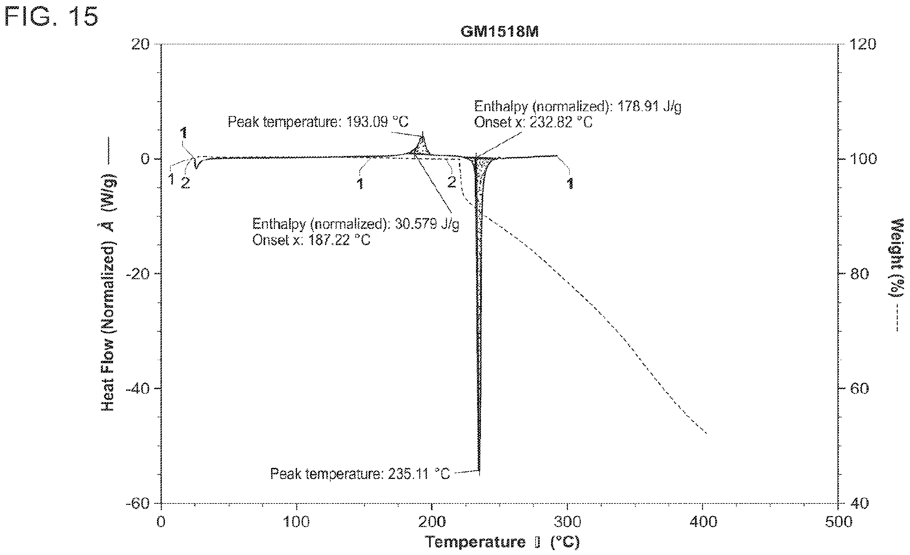

2-Br-LSD free base (100 mg, 0.249 mmol, 1 equiv) was charged to a crystallization tube and toluene (1.0 mL, 10 vol) was added to give a dark orange solution. The mixture was heated to 50 °C and benzenesulfonic acid (1M solution in ethanol, 0.249 mL, 0.249 mmol, 1 equiv) was charged in one portion. The solution was equilibrated at 50 °C for 1 hour, resulting in precipitation of a gummy solid. The mixture was cooled to 25 °C and equilibrated for 24 hours, during which the solid remained gummy. The volume was reduced to 50% under a steady stream of nitrogen, resulting in a free-flowing suspension. The solid was collected by filtration and dried *in vacuo* at 45 °C to give an off-white solid (102 mg, 73% yield). The ¹H NMR spectrum (not shown) shows a significant shift of the N-methyl protons and indicates one molar equivalent of benzenesulfonic acid, confirming the successful formation of a mono besylate salt. The thermal profile (FIG. 15) is consistent with an anhydrous solid with a melt at 193 °C. The XRP diffractogram and peak list are presented as FIG. 8 and Table 7, respectively.

### Example 12: Preparation of crystalline 2-Br-LSD oxalate (Form A)

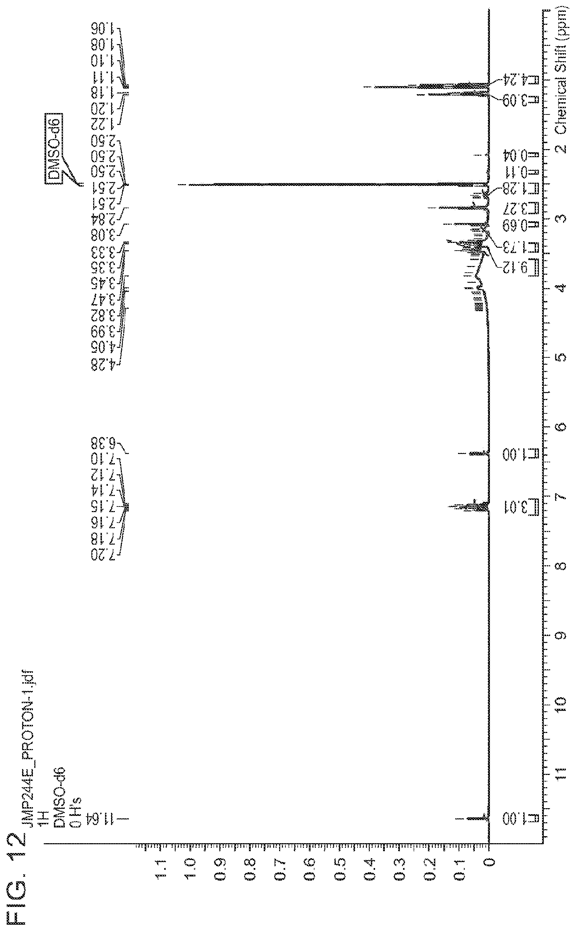

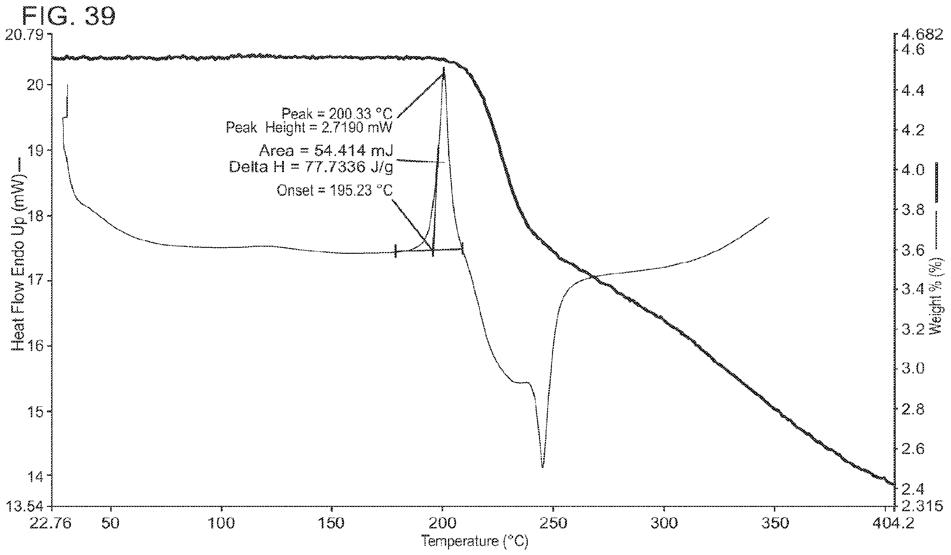

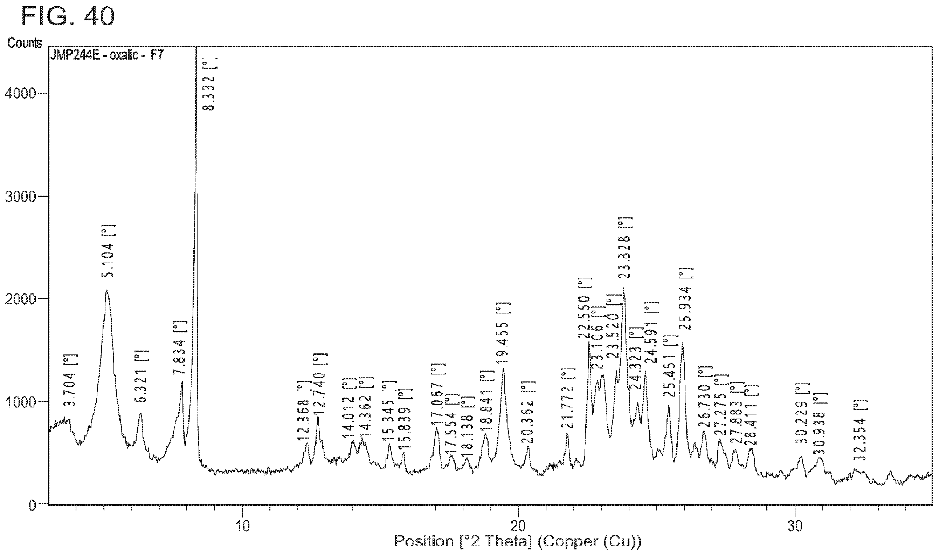

2-Br-LSD free base (30 mg, 0.075 mmol, 1 equiv) was charged to a crystallization tube and ethanol (0.3 mL, 10 vol) was added to give a dark orange solution. The mixture was heated to 50 °C and oxalic acid (1M solution in ethanol, 0.075 mL, 0.075 mmol, 1 equiv) was charged in one portion. The solution was equilibrated at 50 °C for 1 hour, resulting in a solution, and then cooled to 25 °C and equilibrated for 24 hours, resulting in a suspension. The solid was collected by filtration and dried *in vacuo* at 45 °C to give 2-Br-LSD oxalate (Form A) as an off-white solid (7 mg, 19% yield). The ¹H NMR spectrum (FIG. 12) confirmed a successful salt formation due to a shift in the N-methyl signal. The DSC curve (FIG. 39) contained a single endothermic event with peak temperature of 200 °C and an onset of 195 °C. The corresponding TGA curve (FIG. 39) showed no loss of mass prior to the main melt endotherm, indicating an absence of bound water. The XRP diffractogram and peak list are presented as FIG. 40 and Table 4A, respectively.

### Example 13: Preparation of crystalline 2-Br-LSD xinafoate (Form A)

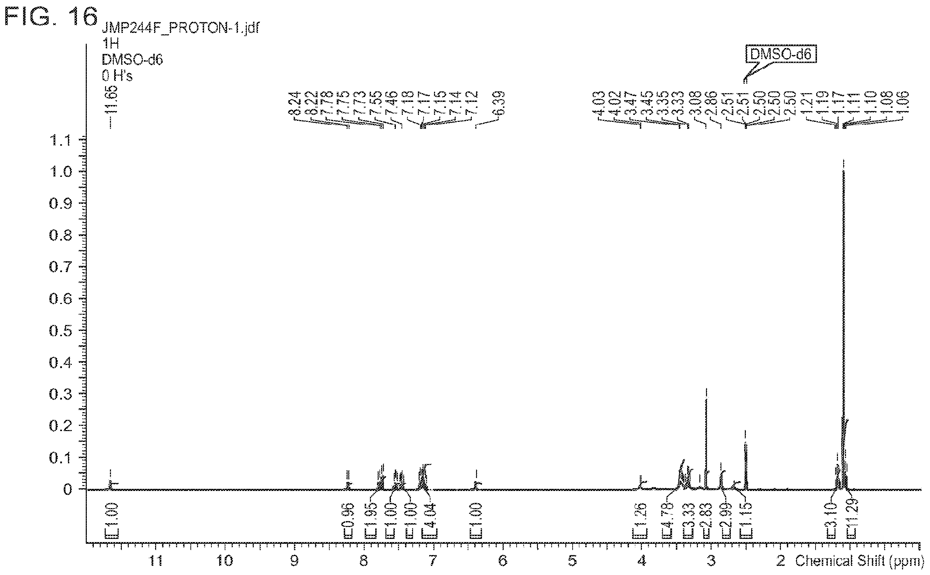

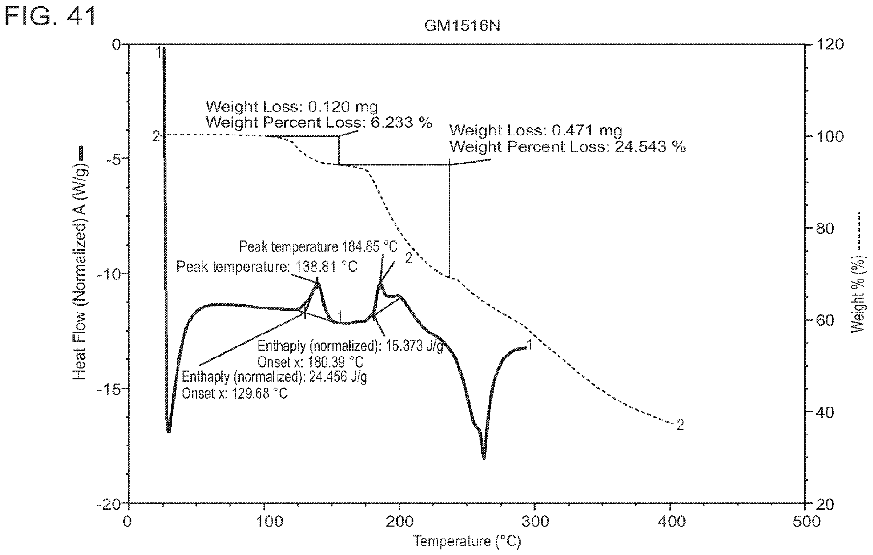

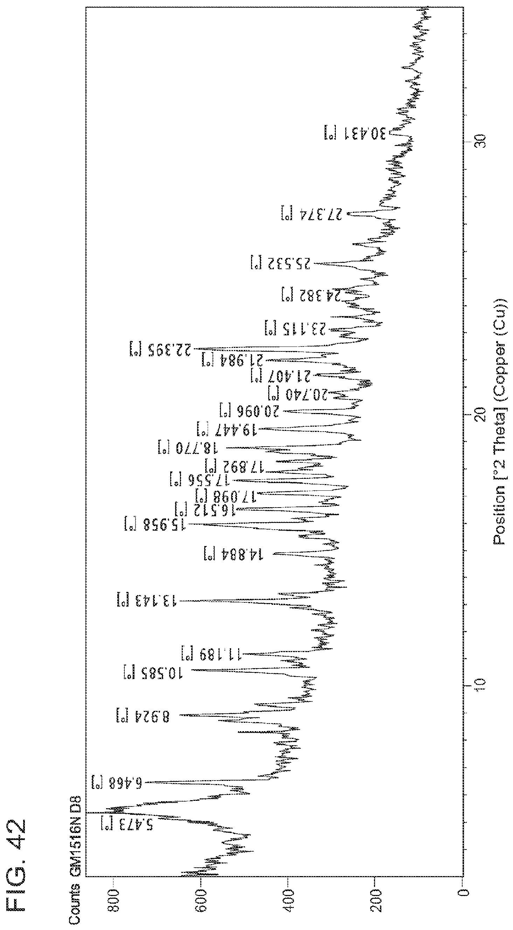

2-Br-LSD free base (30 mg, 0.075 mmol, 1 equiv) was charged to a crystallization tube and ethanol (0.3 mL, 10 vol) was added to give a dark orange solution. The mixture was heated to 50 °C and xinafoic acid (0.5M solution in ethanol, 0.150 mL, 0.075 mmol, 1 equiv) was charged in one portion. The solution was equilibrated at 50 °C for 1 hour, resulting in a solution, and then cooled to 25 °C and equilibrated for 24 hours, resulting in a solution. The solvent was evaporated under a stream of nitrogen, and the sample was triturated with a small amount of methyl tert-butyl ether. The resulting solid was collected by filtration and dried *in vacuo* at 45 °C to give a beige solid (33 mg, 75% yield). The ¹H NMR spectrum (FIG. 16) confirmed the presence of a molar equivalent of xinafoic acid and showed a significant shift of the N-methyl signal, indicating successful salt formation. The DSC curve of the solid (FIG. 41) contained two endotherms at 138 and 185 °C. The first endotherm was associated with a 6% mass loss in the TGA curve (FIG. 41). The NMR spectrum confirmed that ~7% ethanol was present. Therefore, the first endotherm may relate to desolvation, with the true melt of the solid being 184 °C. The XRP diffractogram and peak list are presented as FIG. 42 and Table 3A, respectively.

### Example 14: Preparation of crystalline 2-Br-LSD xinafoate (Form B)

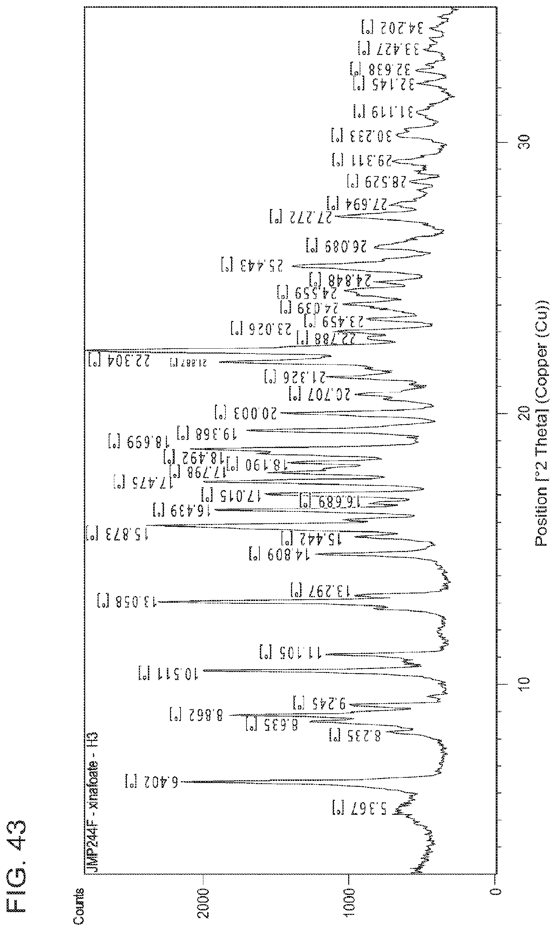

A sample of 2-Br-LSD xinafoate (Form A) was placed in a TA Instruments Discovery DSC and thermally manipulated to 150 °C which was beyond the 6.1% loss shown by TGA analysis and the minor endotherm and exotherm at 129 °C shown by DSC analysis. Following XPRD analysis (FIG. 43 and Table 3B) of the thermally manipulated solid, a version of the xinafoate salt was identified which was designated 2-Br-LSD xinafoate (Form B).

### Example 15: Preparation of crystalline 2-Br-LSD hemi-succinate (Form A)

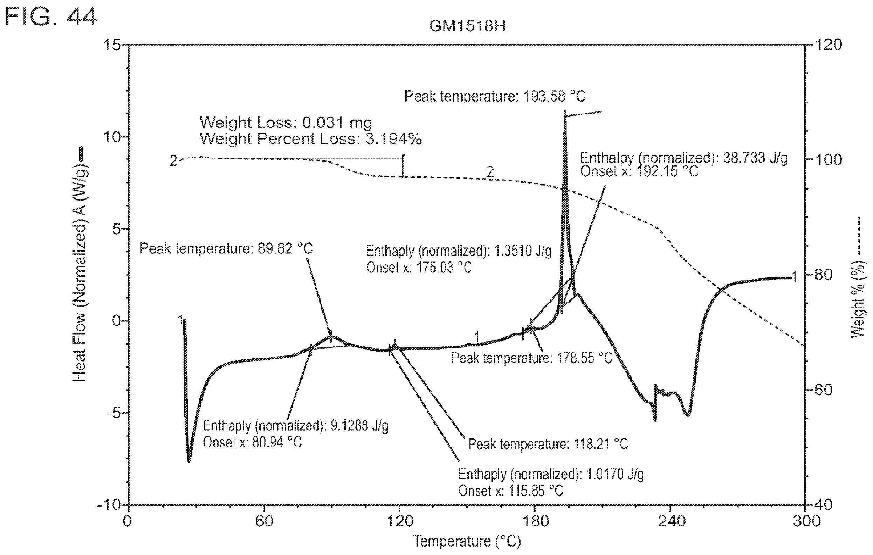

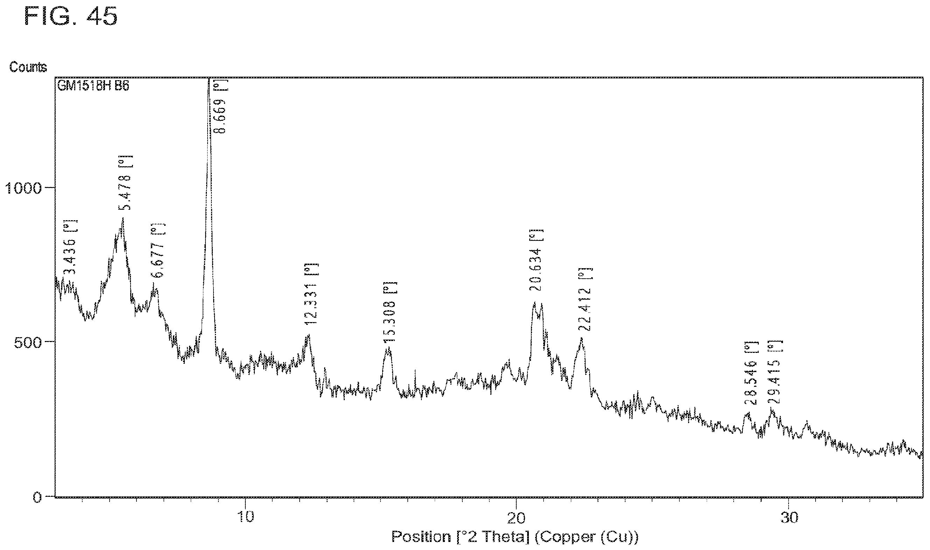

2-Br-LSD free base (100 mg, 0.249 mmol, 1 equiv) was charged to a crystallization tube and ethanol (1.0 mL, 10 vol) was added to give a dark orange solution. The mixture was heated to 50 °C and succinic acid (0.25M solution in ethanol, 0.996 mL, 0.249 mmol, 1 equiv) was charged in one portion. The solution was equilibrated at 50 °C for 1 hour, resulting in a solution, and then cooled to 25 °C and equilibrated for 24 hours, resulting in a solution. The solvent was evaporated under a stream of nitrogen, and the sample was triturated with a small amount of methyl tert-butyl ether. The resulting solid was collected by filtration and dried *in vacuo* at 45 °C to give a beige solid (13 mg, 11% yield). The ¹H NMR spectrum (not shown) suggested a hemi-succinate salt, showing 0.7 molar equivalents of succinic acid and only a minor shift of the N-methyl protons. The DSC curve of the hemi-succinate salt (FIG. 44) showed a main melt endotherm at 196 °C which was preceded by three small endothermic events. The corresponding TGA curve (FIG. 44) showed a 3% weight loss associated with these initial endotherms indicative of solvent and water loss. The XRP diffractogram and peak list are presented as FIG. 45 and Table 6, respectively.

### Example 16: Preparation of crystalline 2-Br-LSD mono-succinate (Form A)

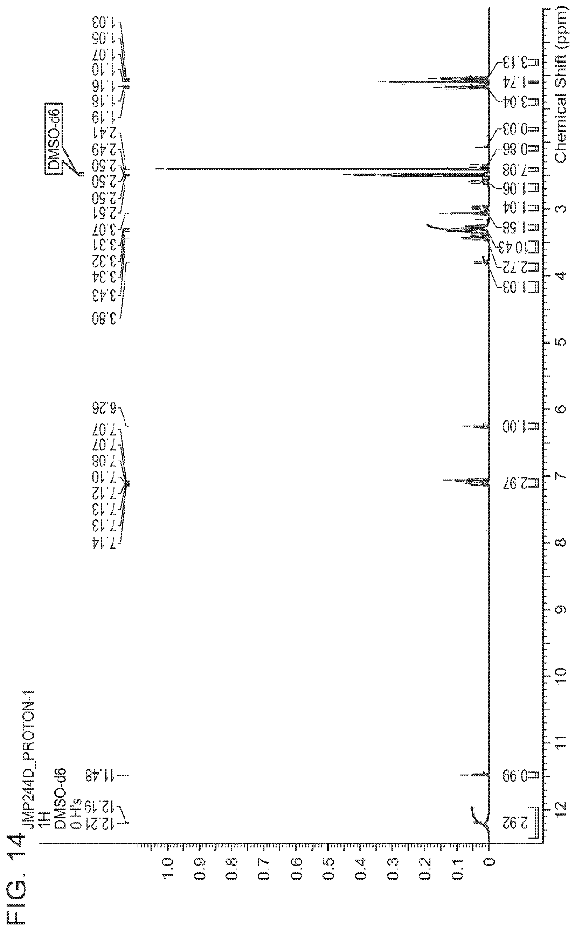

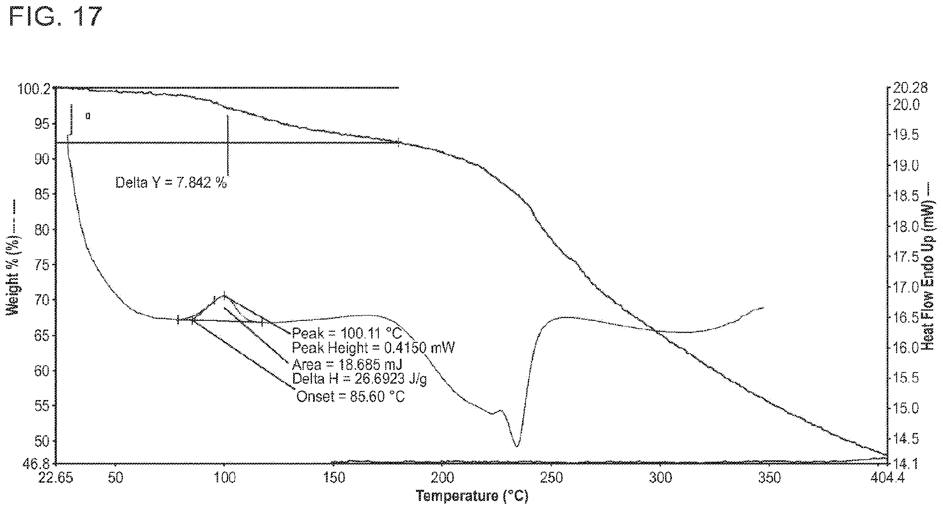

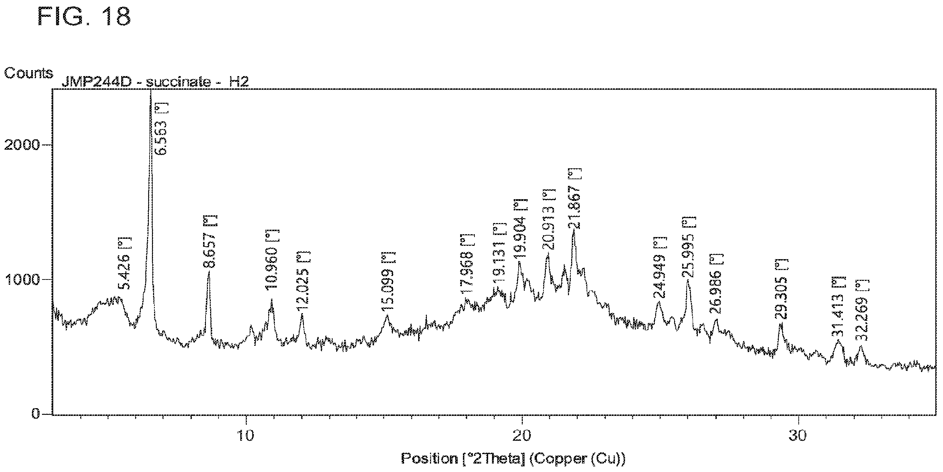

2-Br-LSD free base (100 mg, 0.249 mmol, 1 equiv) was charged to a crystallization tube and toluene (1.0 mL, 10 vol) was added to give a dark orange solution. The mixture was heated to 50 °C and succinic acid (0.25 M solution in ethanol, 0.996 mL, 0.249 mmol, 1 equiv) was charged in one portion. The solution was equilibrated at 50 °C for 1 hour, resulting in a solution, and then cooled to 25 °C and equilibrated for 24 hours, resulting in a solution. The solvent was evaporated under a stream of nitrogen, and the sample was triturated with a small amount of methyl tert-butyl ether. The resulting solid was collected by filtration and dried *in vacuo* at 45 °C to give a faint yellow solid (89 mg, 78% yield). The ¹H NMR spectrum (FIG. 14) confirms a successful salt formation with mono stoichiometry, as a succinic acid signal is present at ~2.4ppm (4H). The XRP diffractogram, XRP peak list, and combined DSC/TGA curves are presented as FIG. 18, Table 8, and FIG. 17, respectively.

All patent publications and non-patent publications are indicative of the level of skill of those skilled in the art to which this disclosure pertains. All these publications (including any specific portions thereof that are referenced) are herein incorporated by reference to the same extent as if each individual publication were specifically and individually indicated as being incorporated by reference.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

### Further Embodiments of the Invention:

The invention may be defined in accordance with any of the following numbered paragraphs.
1. A crystalline salt of 2-bromolysergic acid diethylamide (2-Br-LSD), wherein the salt is a hydrobromide, hemi-fumarate, mesylate, or besylate salt.
2. The crystalline salt of paragraph 1, which is 2-Br-LSD hydrobromide.
3. The crystalline salt of paragraph 2, which has an X-ray powder diffraction (XRPD) pattern comprising the following 2Θ peaks measured using CuK_{α} radiation: 8.0 ± 0.2, 14.2 ± 0.2, 21.9 ± 0.2, 23.3 ± 0.2, 28.6 ± 0.2, 30.4 ± 0.2.
4. The crystalline salt of paragraph 3, which has an XRPD pattern further comprising the following 2Θ peaks measured using CuK_{α} radiation: 12.5 ± 0.2, 17.8 ± 0.2, 19.0 ± 0.2, 24.0 ± 0.2, 24.5 ± 0.2.
5. The crystalline salt of paragraph 4, which has an XRPD pattern further comprising the following 2Θ peaks measured using CuK_{α} radiation: 26.1 ± 0.2, 26.9 ± 0.2.
6. The crystalline salt of paragraph 2 which has an XRPD pattern substantially similar to that set forth in FIG. 1 as measured using CuK_{α} radiation.
7. The crystalline salt of paragraph 2, which has an X-ray powder diffraction (XRPD) pattern comprising the following 2Θ peaks measured using CuK_{α} radiation: 4.4 ± 0.2, 16.9 ± 0.2.
8. The crystalline salt of paragraph 7, which has an XRPD pattern further comprising the following 2Θ peaks measured using CuK_{α} radiation: 7.7 ± 0.2, 15.0 ± 0.2, 16.1 ± 0.2, 18.1 ± 0.2, 19.5 ± 0.2, 23.0 ± 0.2, 24.7 ± 0.2, 25.5 ± 0.2.
9. The crystalline salt of paragraph 2, which has an XRPD pattern substantially similar to that set forth in FIG. 4 as measured using CuKα radiation.
10. The crystalline salt of paragraph 2, which has an X-ray powder diffraction (XRPD) pattern comprising the following 2Θ peaks measured using CuK_{α} radiation: 5.5 ± 0.2, 10.2 ± 0.2, 11.6 ± 0.2, 12.1 ± 0.2, 13.1 ± 0.2.
11. The crystalline salt of paragraph 10, which has an XRPD pattern further comprising the following 2Θ peaks measured using CuK_{α} radiation: 7.5 ± 0.2, 13.5 ± 0.2, 16.3 ± 0.2, 18.9 ± 0.2, 19.4 ± 0.2, 20.5 ± 0.2, 21.2 ± 0.2, 24.1 ± 0.2, 24.6 ± 0.2, 24.9 ± 0.2, 25.2 ± 0.2, 25.7 ± 0.2, 26.3 ± 0.2.
12. The crystalline salt of paragraph 2, which has an XRPD pattern substantially similar to that set forth in FIG. 5 as measured using CuKα radiation.
13. The crystalline salt of paragraph 5, which is non-hygroscopic.
14. The crystalline salt of paragraph 1, which is 2-Br-LSD hemi-fumarate.
15. The crystalline salt of paragraph 14, which has an XRPD pattern comprising the following 2Θ peaks measured using CuK_{α} radiation: 4.4 ± 0.2, 7.4 ± 0.2, 8.3 ± 0.2, 10.3 ± 0.2, 14.9 ± 0.2, 21.9 ± 0.2.
16. The crystalline salt of paragraph 15, which has an XRPD pattern further comprising the following 2Θ peaks measured using CuK_{α} radiation: 9.5 ± 0.2, 11.5 ± 0.2, 11.9 ± 0.2, 13.5 ± 0.2, 16.3 ± 0.2, 17.1 ± 0.2, 18.3 ± 0.2, 19.3 ± 0.2, 19.8 ± 0.2, 20.5 ± 0.2, 20.8 ± 0.2, 21.5 ± 0.2, 22.5 ± 0.2, 23.2 ± 0.2, 23.8 ± 0.2, 25.1 ± 0.2, 26.8 ± 0.2, 28.7 ± 0.2, 30.1 ± 0.2, 30.6 ± 0.2.
17. The crystalline salt of paragraph 14, which has an XRPD pattern substantially similar to that set forth in FIG. 6. as measured using CuKα radiation.
18. The crystalline salt of paragraph 1, which is 2-Br-LSD mesylate.
19. The crystalline salt of paragraph 18, which has an XRPD pattern comprising the following 2Θ peaks measured using CuK_{α} radiation: 7.5 ± 0.2.
20. The crystalline salt of paragraph 19, which has an XRPD pattern further comprising the following 2Θ peaks measured using CuK_{α} radiation: 7.2 ± 0.2, 10.1 ± 0.2, 10.8 ± 0.2, 13.3 ± 0.2, 15.1 ± 0.2, 15.5 ± 0.2, 17.2 ± 0.2, 19.4 ± 0.2, 20.5 ± 0.2, 21.6 ± 0.2, 22.3 ± 0.2, 23.2 ± 0.2, 23.5 ± 0.2, 24.4 10.2, 26.1 ± 0.2, 28.1 ± 0.2.
21. The crystalline salt of paragraph 18, which has an XRPD pattern substantially similar to that set forth in FIG. 7 as measured using CuKα radiation.
22. The crystalline salt of paragraph 1, which is 2-Br-LSD besylate.
23. The crystalline salt of paragraph 22, which has an XRPD pattern comprising the following 2Θ peaks measured using CuK_{α} radiation: 5.9 ± 0.2, 8.7 ± 0.2, 17.8 ± 0.2, 19.7 ± 0.2, 23.4 ± 0.2.
24. The crystalline salt of paragraph 23, which has an XRPD pattern further comprising the following 2Θ peaks measured using CuK_{α} radiation: 13.4 ± 0.2, 13.8 ± 0.2, 14.6 ± 0.2, 16.1 ± 0.2, 16.3 ± 0.2, 16.8 ± 0.2, 20.5 ± 0.2, 21.6 ± 0.2, 22.3 ± 0.2, 22.9 ± 0.2, 23.8 ± 0.2, 24.5 ± 0.2.
25. The crystalline salt of paragraph 22, which has an XRPD pattern substantially similar to that set forth in FIG. 8 as measured using CuKα radiation.
26. A pharmaceutical composition comprising the crystalline salt of paragraph 1.
27. A pharmaceutical composition comprising the crystalline salt of paragraph 5.
28. A method of preparing the crystalline salt of 2-Br-LSD hydrobromide (Form 1), which has an XRPD pattern substantially similar to that set forth in FIG. 1 as measured using CuKα radiation, comprising the following steps:
   (a) equilibrating a solution comprising 2-Br-LSD and a solvent at a first temperature;
   (b) adding a first portion of hydrobromic acid to the first-temperature equilibrated solution;
   (c) equilibrating the batch at the first temperature for a period of time M;
   (d) adding a second portion of hydrobromic acid to the batch over a period of time N;
   (e) equilibrating the batch at the first temperature for a period of time O;
   (f) cooling the batch to a second temperature;
   (g) equilibrating the batch at the second temperature for a period of time P;
   (h) isolating precipitate from the suspension; and
   (i) drying the isolated precipitate *in vacuo* at a third temperature to obtain the crystalline salt;
      wherein M is between 0 and 24 hours;
      wherein N is between 5 minutes and 24 hours;
      wherein O is between 0 and 8 hours;
      wherein P is between 30 minutes and 48 hours.
29. The method of paragraph 28, further comprising adding seed crystals of 2-Br-LSD hydrobromide (Form 1) to the batch prior to step (c).
30. The method of paragraph 28, wherein the solvent comprises water and ethanol, methanol or isopropanol.
31. The method of paragraph 30, wherein the solvent comprises 2-12% water.
32. The method of paragraph 31, wherein the solvent comprises ethanol.
33. The method of paragraph 32, wherein the solvent comprises 88% to 98% ethanol.
34. The method of paragraph 33, wherein the solvent is ethanol-water in a ratio of about 95:5 v/v.
35. The method of paragraph 34, wherein the concentration of 2-Br-LSD in step (a) is between 0.12M and 0.6M.
36. The method of paragraph 35, wherein the concentration of 2-Br-LSD in step (a) is between 0.2M and 0.4M.
37. The method of paragraph 28, wherein the hydrobromic acid is added as a solution.
38. The method of paragraph 37, wherein the hydrobromic acid is added as a solution in the same solvent used in step (a).
39. The method of paragraph 38, wherein hydrobromic acid is added as a solution in ethanol-water at a concentration of 0.5M to 4M.
40. The method of paragraph 33, wherein hydrobromic acid is added as a solution in the same solvent used in step (a) at a concentration of 1M to 3M.
41. The method of paragraph 28, wherein the first temperature is between about 20 °C and 50 °C.
42. The method of paragraph 41, wherein the first temperature is between 30 °C and 50 °C and the second temperature is between 0 °C and about 25 °C.
43. The method of paragraph 42, wherein the first temperature is between 40 °C and 50 °C and the second temperature is about 20 °C to about 25 °C.
44. The method of paragraph 43, wherein M is between 1 and 4 hours, N is between 2 and 6 hours, O is between 30 minutes and 4 hours, and P is between 8 and 24 hours.
45. The method of paragraph 44, further comprising:
   following period P and prior to step (h), cooling the batch to a fourth temperature and equilibrating the batch at the fourth temperature for a period of time Q;
   wherein Q is between 5 minutes and 48 hours.
46. The method of paragraph 45, wherein the fourth temperature is between 0 °C and 10 °C.
47. The method of paragraph 46, wherein Q is between 0.5 hour and 4 hours.
48. The method of paragraph 42, wherein the first temperature is between about 20 °C and about 25 °C and the second temperature is between 0 °C and 10 °C.
49. The method of paragraph 48, wherein M is between 1 and 4 hours, N is between 2 and 6 hours, O is between 30 minutes and 4 hours, and P is between 8 and 24 hours.
50. The method of paragraph 28, wherein the sum of the hydrobromic acid portions in steps (b) and (d) is 1.0 molar equivalent with respect to 2-Br-LSD.
51. The method of paragraph 50, wherein the portion of hydrobromic acid in step (b) is sufficient to induce the formation of crystals of 2-Br-LSD hydrobromide.
52. The method of paragraph 51, wherein the addition of hydrobromic acid in step (b) is halted at the first visible sign of crystallization.
53. The method of paragraph 52, further comprising adding seed crystals of 2-Br-LSD hydrobromide (Form 1) to the batch prior to step (c).
54. The method of paragraph 28, wherein hydrobromic acid is added as a gas in steps (b) and (d).
55. The method of paragraph 50, wherein M is 0.
56. The method of paragraph 50, wherein the hydrobromic acid portion in step (b) is between 0.1 and 0.4 molar equivalents with respect to 2-Br-LSD.
57. The method of paragraph 56, wherein the hydrobromic acid portion in step (b) is between 0.2 and 0.3 molar equivalents with respect to 2-Br-LSD.

## Claims

1. A crystalline salt of 2-bromolysergic acid diethylamide (2-Br-LSD), which is 2-Br-LSD hydrobromide.

2. The crystalline salt of claim 1, which has an X-ray powder diffraction (XRPD) pattern comprising the following 2Θ peaks measured using CuK_{α} radiation: 8.0 ± 0.2, 14.2 ± 0.2, 21.9 ± 0.2, 23.3 ± 0.2, 28.6 ± 0.2, 30.4 ± 0.2;
wherein optionally the crystalline salt has an XRPD pattern further comprising the following 2Θ peaks measured using CuK_{α} radiation: 12.5 ± 0.2, 17.8 ± 0.2, 19.0 ± 0.2, 24.0 ± 0.2, 24.5 ± 0.2; and
wherein optionally the crystalline salt has an XRPD pattern further comprising the following 2Θ peaks measured using CuK_{α} radiation: 26.1 ± 0.2, 26.9 ± 0.2.

3. The crystalline salt of claim 1, which is non-hygroscopic.

4. The crystalline salt of claim 3, wherein non-hygroscopic is determinable by either:
a crystalline salt that, after drying *in vacuo* at 50 °C for 18 hours, exhibits less than 1% weight loss between 25 °C and 200 °C upon thermogravimetric analysis at a heating rate of 20 °C/min; or
a crystalline salt exhibits less than about 0.5% weight change upon dynamic vapor sorption analysis at 25 °C over the relative humidity (RH) range 0-90% when measured at 10% RH intervals and 60 minutes for each humidity level.

5. A pharmaceutical composition comprising the crystalline salt of any of claims 1 to 4.

6. The pharmaceutical composition of claim 5, further comprising one or more pharmaceutically acceptable excipients.

7. The pharmaceutical composition of claim 5 or 6, wherein the pharmaceutical composition is a solid composition for oral administration.

8. The pharmaceutical composition of claim 7, wherein the pharmaceutical composition comprises between 1.5 mg and 5 mg of the crystalline salt of claim 1.

9. A method of preparing a crystalline salt of 2-Br-LSD hydrobromide, comprising the following steps:
(a) equilibrating a solution comprising 2-Br-LSD and a solvent at a first temperature;
(b) adding a first portion of hydrobromic acid to the first-temperature equilibrated solution;
(c) equilibrating the batch at the first temperature for a period of time M;
(d) adding a second portion of hydrobromic acid to the batch over a period of time N;
(e) equilibrating the batch at the first temperature for a period of time O;
(f) cooling the batch to a second temperature;
(g) equilibrating the batch at the second temperature for a period of time P;
(h) isolating precipitate from the suspension; and
(i) drying the isolated precipitate *in vacuo* at a third temperature to obtain the crystalline salt;
wherein M is between 0 and 24 hours;
wherein N is between 5 minutes and 24 hours;
wherein O is between 0 and 8 hours;
wherein P is between 30 minutes and 48 hours.

10. The method of claim 9, further comprising adding seed crystals of 2-Br-LSD hydrobromide (Form 1), which has an X-ray powder diffraction (XRPD) pattern comprising the following 2Θ peaks measured using CuK_{α} radiation: 8.0 ± 0.2, 14.2 ± 0.2, 21.9 ± 0.2, 23.3 ± 0.2, 28.6 ± 0.2, 30.4 ± 0.2, to the batch prior to step (c).

11. The method of claim 9, wherein the solvent comprises 2-12% water.

12. The method of claim 11, wherein the solvent comprises 88% to 98% ethanol.

13. The method of claim 9, wherein the first temperature is between about 20 °C and 50 °C.

14. The method of claim 13, wherein the first temperature is between 30 °C and 50 °C and the second temperature is between 0 °C and about 25 °C.

15. The method of claim 9, wherein the sum of the hydrobromic acid portions in steps (b) and (d) is 1.0 molar equivalent with respect to 2-Br-LSD.
